Europäisches Patentamt

European Patent Office

Office européen des brevets

(11) Publication number: **0 261 648 B1**

## EUROPEAN PATENT SPECIFICATION

(45) Date of publication of patent specification: **09.12.92**

(51) Int. Cl.5: **A61K 35/26**, A61K 37/02, A61K 39/00

(21) Application number: **87113856.6**

(22) Date of filing: **22.09.87**

(54) Cell membrane proteins, compositions containing them and procedure for their preparation.

(30) Priority: **23.09.86 US 910876**

(43) Date of publication of application:
**30.03.88 Bulletin 88/13**

(45) Publication of the grant of the patent:
**09.12.92 Bulletin 92/50**

(84) Designated Contracting States:
**AT BE CH DE ES FR GB GR IT LI LU NL SE**

(56) References cited:
**GB-A- 2 146 338**

**BIOLOGICAL ABSTRACT, vol. 80, no. 2, 1985, abstract 9900 C. PASTERNAK et al.: "Lymphocyte mechanical response triggered by cross-linking surface receptors"**

**CHEMICAL ABSTRACTS, vol. 82, no. 11, March 17, 1975, page 320, abstract 71485r Columbus, Ohio, US J.M. LANG et al.: "High hydrostatic pressures and lymphocyte reactivity to phytohemagglutinin stimulation"**

(73) Proprietor: **YEDA RESEARCH AND DEVELOPMENT COMPANY, LTD.**
**Kiryat Weizman P.O. Box 95**
**Rehovot 76100(IL)**

(72) Inventor: **Cohen, Irun Robert**
**11 Hankin Street**
**Rehovot 76100(IL)**
Inventor: **Shinitzky, Meir**
**Meonot Wolfson Apt. D33**
**Institute of Science Rehovot 76100(IL)**

(74) Representative: **Henkel, Feiler, Hänzel & Partner**
**Möhlstrasse 37**
**W-8000 München 80(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

CHEMICAL ABSTRACTS, vol. 79, no. 15, October 15, 1973, page 70, abstract 87915e Columbus, Ohio, US M. ONO et al.: "Effect of cytochalasin B on lymphocyte stimulation induced by concanavalin A or periodate"

CHEMICAL ABSTRACTS, vol. 73, no. 19, November 9, 1970, page 239, abstract 97235y Columbus, Ohio, US D.A. KILLEN: "In vivo response of mouse lymphocytes to phytohemagglutinin and colchicine"

JOURNAL OF IMMUNOLOGY, vol. 131, no. 6, December 1983, pages 2810-2813; The American Ass. of Imm., US J. HOLOSHITZ et al.: "Autoimmune encehalomyelitis (EAE) mediated or prevented by T lymphocyte lines directed against diverse antigenic determinants of myelin basic protein. Vaccination is determinant specific"

JOURNAL OF IMMUNOLOGY, vol. 131, no. 5, November 1983, pages 2316-2322, The American Ass. of Imm., US R. MARON et al.: "T lymphocyte line specific for thyroglobulin produces or vaccinates against autoimmune thyroiditis in mice"

JOURNAL OF IMMUNOLOGY, vol. 121, no. 5, November 1978, pages 1760-1766, Williams and Wilkins Co., US D.W. THOMAS: "Hapten.specific T lymphocyte activation by glutaraldehyde-treated macrophages: An argument against antigen processing by macrophages"

PROCEEDINGS OF THE NATIONAL ACADEMY OF SCIENCES USA, vol. 84, no. 13, July 1978, pages 4577-80 O. LIDER et al.: "Therapeutic vaccination against adjuvant arthritis using autoimmune T cells treated with hydrostatic pressure"

ANNALS OF THE NEW YORK ACADEMY OF SCIENCES, vol. 0, no. 0, 1986, pages 267-273, US O. LIDER et al.: "Vaccination against experimental autoimmune diseases using T lymphocytes treated with hydrostatic pressure"

**Description**

Within this application several publications are referenced by Arabic numerals within parentheses. Full citations for these references may be found at the end of the specification immediately preceding the claims.

The invention relates to novel preparations and drugs for the treatment and prevention of autoimmune diseases, which are based on immunogenic materials obtained from membranes of certain cells and cell lines of autoimmune lymphocytes or which are based on activated cells which have been treated with a novel pressure treatment, a chemical cross-linking agent, or a cytoskeletal disrupting agent.

The preparations for the prevention of autoimmune diseases include vaccines which comprise membrane proteins obtained from specific autoimmune T cell lines containing certain T cell receptors or pressure-treated activated T cells. The invention further provides processes for the preparation of such active cell membrane materials and for the treatment of activated cells, and to pharmaceutical compositions which contain either the cell membrane materials or the activated cells as their active ingredient.

The etiological agents of autoimmune diseases are endogenous lymphocytes which attack normal constituents of the individual. The inventors have been engaged in growing as long term cell lines specific autoimmune T lymphocytes which produce a number of experimental autoimmune diseases (1-9). The thus obtained comparatively pure cultures of autoimmune cells have facilitated investigation of pathogenesis, uncovered the carrier state of autoimmunity and provided means for vaccination against, and treating, autoimmunity (5-9).

The present invention relates to novel preparations for use in the prevention and treatment of autoimmune diseases, said preparations having as their active ingredient certain membrane materials of specific autoimmune T lymphocytes or such pressure treated-activated T lymphocyte cells. The invention further relates to a process for obtaining such materials from such lymphocytes, to the pressure treatment of such lymphocytes and for the preparation of vaccines and pharmaceutical preparations containing same. Other and further features of the invention will become apparent hereinafter.

Autoimmune diseases share the common feature of being caused by the immune system attacking an individual's own tissues. At the seat of all autoimmune diseases are the autoimmune lymphocytes which specifically recognize the individual's particular target antigens. Amongst autoimmune diseases there may be mentioned rheumatoid arthritis, multiple sclerosis, some forms of diabetes mellitus, thyroiditis, and myasthenia gravis. Hitherto there has not existed any specific form of therapy against these diseases.

It has been found possible to grow as long term cell lines T lymphocytes responsible for causing autoimmune diseases in laboratory animals. Amongst such diseases there may be mentioned encephalomyelitis, arthritis and thyroiditis. Such cells were found to be effective agents for vaccination against such specific autoimmune diseases: such lymphocytes were attenuated and injected so that they would not cause the autoimmune diseases. It was found that such vaccinations were quite effective in rendering such animals immune to, or less sensitive (the disease was much less severe) to such diseases. Furthermore, it was shown that when such animals were inoculated with such cells, this constituted a quite effective treatment of the disease.

According to the present invention there are provided pharmaceutical preparations which contain as their active ingredient membrane material from specific autoimmune T cells which contain certain T cell receptors. The invention also provides a novel process for the production and isolation of such membrane material which comprises exposing such T lymphocytes to a high hydrostatic pressure and gradually releasing the pressure, thus resulting in an effective shedding of membrane material which retains a high degree of biological activity. An alternative is the pressure treatment of such T cells by subjecting them to hydrostatic pressure, and releasing such pressure in a gradual manner.

Typical conditions for the shedding of the active material are pressure of the order of 50.7 to 152 MPa (500 to 1500 atmospheres), the build up of pressure being gradually over 5 minutes, maintaining such pressure at the upper level for about 10 to 45 minutes, and gradually releasing the pressure over 5 to 15 minutes.

Pressure treatment of activated T cells is attained by subjecting such cells to a build up of pressure during about 5 minutes, going up from 500 to about 1300 atmospheres, maintaining the pressure for about 15 minutes and gradually releasing the pressure during about 5 minutes.

Practically no shedding takes place. Both the shed material as well as the pressure-treated, activated cells may be used as active ingredients in the vaccines of the present invention: the cells retain their full viability.

The materials thus obtained comprise about $10^7$ to $10^8$ activated cells or the material from an equal number of cells. For vaccination of humans there is used a quantity of the order of 0.01 mg to 3 mg of such

3

EP 0 261 648 B1

materials (shed protein or pressure-treated, activated T cells), vaccination being given 3-4 times with an interval of about 2 weeks between applications.

Such immunizations are effective for the prevention and treatment of certain autoimmune diseases.

The pressure treatment comprises suspending lymphocytes in a suitable buffer, inserting the suspension into a pressure vessel devoid of any gaseous medium and applying pressure, e.g. with a French press, as set out above.

The resulting cell suspension is subjected to centrifugation at about 1500 rpm and the supernatant is subjected to ultracentrifugation at about 100,000 g for about 1 hour to precipitate the membrane fragments. When pressure-treated, activated T cells are prepared, these are collected by centrifugation.

According to a further embodiment of the invention, purified membrane proteins from T lymphocyte cells useful for preventing or treating a specific autoimmune disease may be produced by subjecting such cells to a treatment which results in the formation of stable aggregates of cell membrane glycoproteins. It is believed that such aggregates include T cell receptors, probably in combination with one or more unidentified molecules which appear after activation of the T cells by treatment with a mitogen or an antigen.

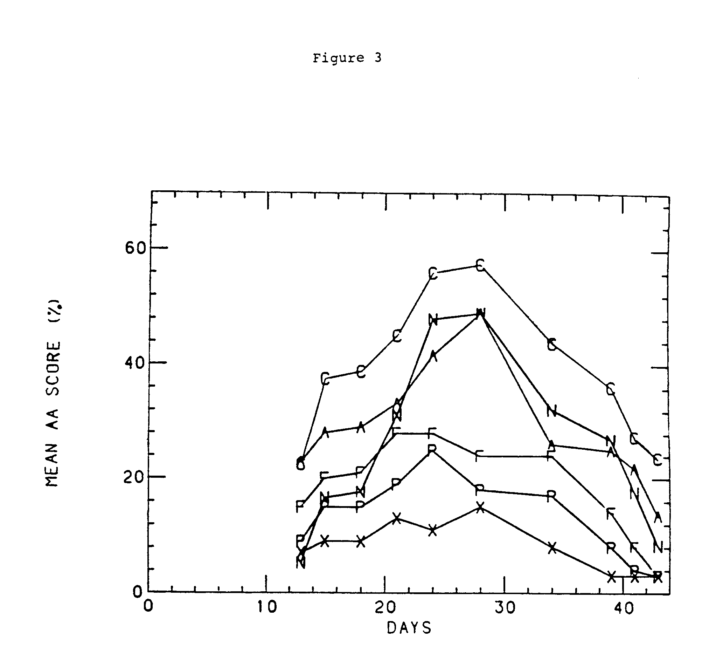

Instead of subjecting the T cells to a pressure treatment, they may be subjected to a treatment with a suitable chemical cross-linking agent, such as formaldehyde, glutaraldehyde or any other conventional cross-linking agent (see Figures 3, 4 and 5). Such treatment is also of value when applied prior or subsequent to the pressure treatment.

According to yet a further embodiment of the invention, such T cells may be subjected to a treatment adapted to disrupt the cytoskeleton of said T lymphocytes. Conventional cytoskeletal disrupting agents, such as cytochalsin (0.5 micrograms/ml) or colchicine (10 micromolar) may be used.

When chemical cross-linking agents of the type defined above are used, such treatment makes possible the use of isolated cell membranes which are prevented from disintegration after disruption of the cells. While presure treated cells lose some of their vaccination effectiveness after lysing of the cells, cells subjected to chemical cross-linking do not lose such effectiveness.

According to the above, a variety of treatments of T lymphocyte cells, or a combination of such treatments may be used in order to obtain effective means for the prevention and treatment of certain diseases.

The cross-linking of membrane proteins is advantageously effected by treatment with an agent like formaldehyde or glutaraldehyde (about 0.3 to 1 percent for a period of time of 15 minutes).

The disruption of the cytoskeleton may be effected by treatment with an agent such as a combination of cytochalasin (0.5 micrograms/ml) and colchicine (10 micromolar).

After cross-linking, the fixed cell membranes may be obtained by suspending the cells in a suitable hypotonic solution, homogenizing and isolating them using a discontinuous sucrose gradient, followed by dialysis of the separated membranes against phosphate buffered saline (PBS).

The following experiments illustrate some of the results obtained by treating T lymphocyte cells according to the methods described above. The details of the experiments are as follows:

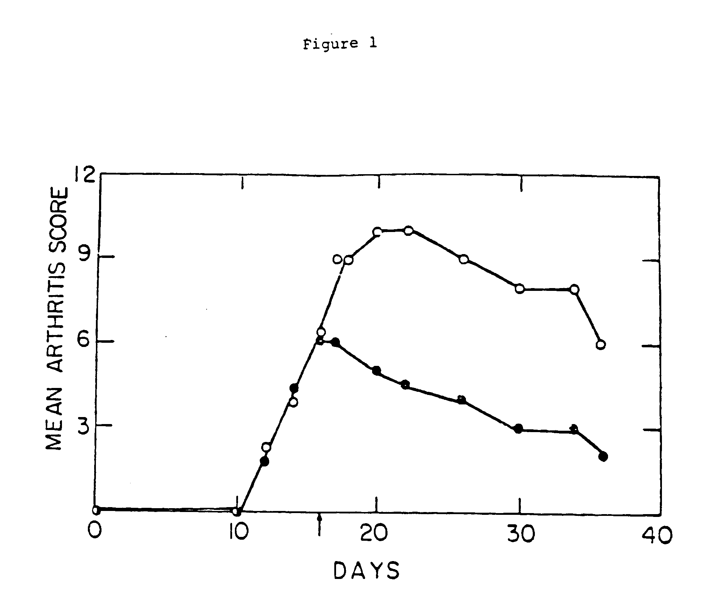

Fig. 1 Treatment of ongoing arthritis by line cells.

Active adjuvant arthritis was induced in 20 Lewis rats by inoculation of CFA. Five days after the onset of clinical arthritis (arrow, day 16), one group of 10 rats was treated by a single intravenous inoculation of activated anti-Tb line cells (closed circles). A second group of 10 rats were treated with an irrelevant control line (open circles). The mean arthritis score was determined as described in (5).

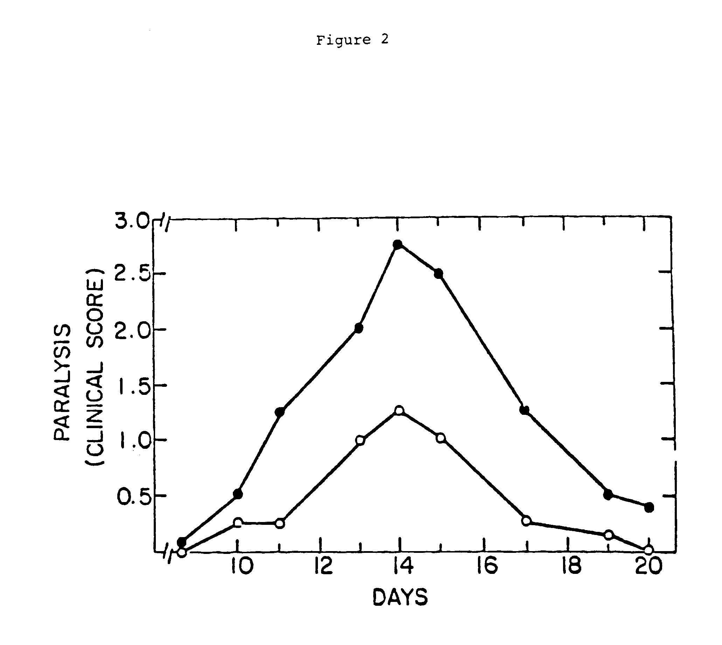

Fig. 2 Alleviation of EAE by administration of membrane fraction.

Rats were inoculated twice at a one week interval with membrane fractions of Z1a line cells (0.5 micrograms obtained by pressure method) and two weeks later challenged with an encephalogenic dose of EAE. The clinical score of the test rats is indicated by the open circles and the control rats by the closed circles. Clinical score: 1 - mild; 2 - moderate; 3 - severe.

Fig. 3 Inhibition of adjuvant arthritis by T lymphocyte vaccines.

Adjuvant athritis was induced in groups of 10 Lewis rats by immunizing them intradermally at the base of the tail with Mycobacterium tuberculosis (MT) H37RA (1 gm) in oil. The severity of disease was graded on a scale of 0 to 100% based on swelling, redness, deformity and lack of function of the paws. The groups were as follows:

C - control, no vaccination.

A - vaccinated before induction of arthritis with 3 weekly injections subcutaneously with $20 \times 10^6$ cells of the clone A2b, that had been activated by incubation for 3 days with the T cell mitogen concanavalin A (1.5 micrograms/ml).

4

N - vaccinated with A2b cells that were not activated but were treated with formaldehyde (0.3% for 15 min).

F - vaccinated with activated A2b cells that were treated with formaldehyde.

P - vaccinated with activated A2b cells that were treated with hydrostatic pressure (1250 bars, 15 min).

X - vaccinated with activated A2b cells that were first treated with pressure and then with formaldehyde.

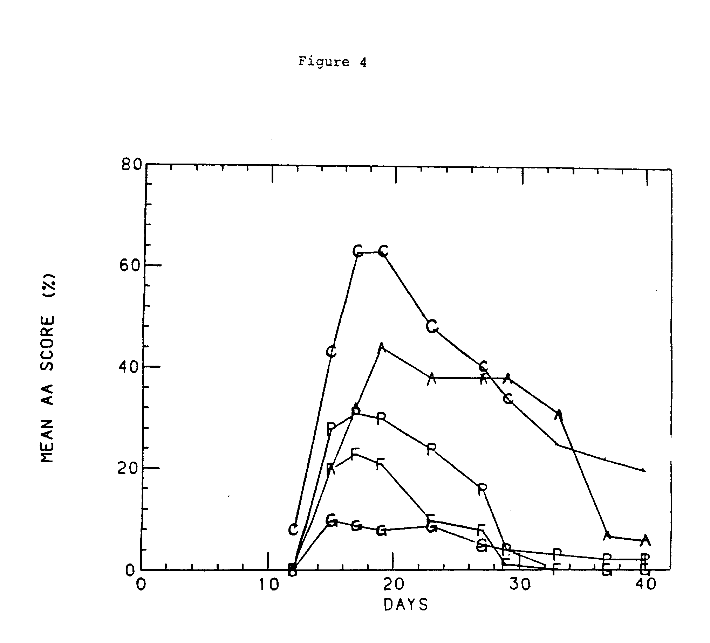

Fig. 4    Inhibition of adjuvant arthritis by T lymphocyte vaccines.

The experiment was performed as described in the description of Figure 3 and the groups have the same labels except that F here signifies  treatment of the A2b cells with formaldehyde (0.3%) after treatment with pressure (1500 bars) and G signifies treatment of the A2b cells with glutaraldehyde (0.3%) after treatment with pressure.

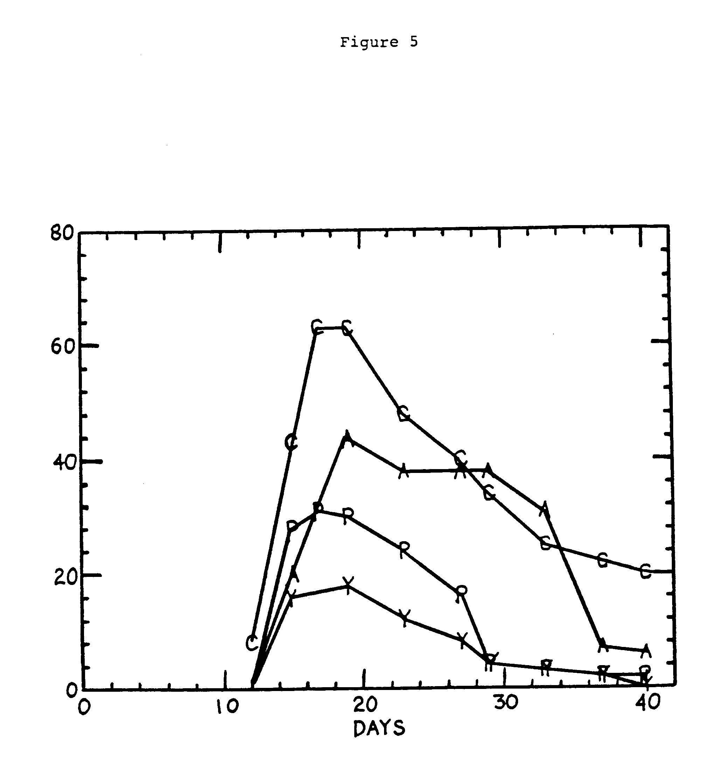

Fig. 5    Inhibition of adjuvant arthritis by a T lymphocyte vaccine prepared with cytoskeletal disruptors.

The experiment was performed as described in the description of Fig. 3. Y signifies A2b cells that had been treated with colchicine (10 micromolar) plus cytochalasin (0.5 micrograms/ml) for 15 min before vaccination.

The present invention provides an optionally pressure-treated, activated T lymphocyte cell useful for preventing or treating a specific autoimmune disease, which additionally has been treated with a chemical cross-linking agent or a cytoskeletal disrupting agent or both. The T lymphocyte cells of the present invention may be derived from an established cell line or may be taken from the circulatory or lymphatic systems of a subject, e.g. a mouse, a rat or a human. Additionally, the T lymphocyte cells may be taken directly for a patient who is to be treated for a specific autoimmune disease. Within this application, "activated T lymphocyte cell" means a T lymphocyte cell which has been exposed to a specific antigen or mitogen capable of inducing an immune response by the T lymphocyte cell. Suitable mitogens are known in the art and include concanavalin A, phytohemagglutinin, and pokeweed mitogen. The pressure-treated and chemically cross-linked or disrupted, activated T lymphocyte cells of the present invention may be useful for treating multiple sclerosis, thyroiditis, diabetes Type I, ankylosing spondylitis, rheumatoid arthritis, or myasthenia gravis.

Suitable chemical cross-linking agents are known in the art and include, but are not limited to, formaldehyde and glutaraldehyde. In one embodiment of the invention, the pressure-treated, activated T lymphocyte cells are treated with the chemical cross-linking agent after being treated with hydrostatic pressure. In another embodiment of the invention, the pressure-treated, activated T lymphocyte cells are treated with the chemical cross-linking agent prior to being treated with hydrostatic pressure.

Within this application, a disrupting agent means an agent capable of causing the cytoskeleton to disassociate into fragments and includes the chemicals cytochalasin and colchicine. However, other disrupting agents are known in the art and may also be used. These disrupting agents may be used individually or in combination with each other.

The disrupting agent-treated, activated T lymphocyte cells of the present invention may additionally be treated with a chemical cross-linking agent or with hydrostatic pressure.

Further provided is a method for producing pressure-treated, activated T lymphocytes useful for preventing or treating a specific autoimmune disease. This method comprises initially suspending activated T lymphocyte cells specific for the autoimmune disease in a buffer. The suspended cells are then subjected to a suitable hydrostatic pressure for an appropriate period of time. Thereafter, the pressure on the suspended cells is released at an appropriate rate so as to produce pressure-treated, activated T lymphocyte cells without substantial loss of membrane proteins from the cells. In one embodiment of the invention, the suitable pressure is from 50.7 to 152 MPa (500 to 1500 atmospheres). In another embodiment of the invention, prior to subjecting the suspended cells to a suitable hydrostatic pressure, the suspended cells are treated with a chemical cross-linking agent. In yet a further embodiment of the invention, the suspended cells are treated with a chemical cross-linking agent after having been depressurized.

Also provided is a method for producing chemical cross-linking agent-treated, activated T lymphocytes useful for preventing or treating a specific autoimmune disease. This method comprises suspending activated T lymphocyte cells specific for the autoimmune disease in a buffer and treating the suspended cells with a chemical cross-linking agent.

The present invention further provides a method for producing pressure-treated, activated T lymphocyte cell membrane fragments useful for preventing or treating a specific autoimmune disease. This method comprises initially suspending activated T lymphocyte cells specific for the autoimmune disease in a buffer. The suspended cells are treated with a disrupting agent so as to obtain activated T lymphocyte cells. The activated T lymphocyte cells are then subjected to a suitable hydrostatic pressure for an appropriate period of time. Thereafter, the pressure on the activated T lymphocyte cell membrane fragments is released at an appropriate rate so as to produce pressure-treated, activated T lymphocyte cell membrane fragments without substantial loss of membrane proteins. In one embodiment of the invention, the suitable pressure is from 500 to about 1300 atmospheres. In another embodiment of the invention, the activated T lymphocyte cell membrane fragments are treated with a chemical cross-linking agent after having been treated with the disrupting agent.

Further provided is another method for producing pressure-treated, activated T lymphocyte cell membrane fragments useful for preventing or treating a specific autoimmune disease. This method comprises initially suspending activated T lymphocyte cells specific for the autoimmune disease in a buffer. The suspended cells are subjected to a suitable hydrostatic pressure for an appropriate period of time. Thereafter, the pressure on the suspended cells is released at an appropriate rate so as to produce pressure-treated, activated T lymphocyte cells without substantial loss of membrane proteins. After having been depressurized, the pressure-treated, activated T lymphocyte cells are treated with a disrupting agent so as to produce pressure-treated, activated T lymphocyte cells. In one embodiment of the invention, the suitable pressure is from 50.7 to 152 MPa (500 to 1500 atmospheres). In yet another embodiment of the invention, the resulting pressure-treated, activated T lymphocyte cells may be treated with a chemical cross-linking agent.

The present invention also provides a method for producing activated T lymphocyte cell membrane fragments useful for preventing or treating a specific autoimmune disease. The method comprises suspending activated T lymphocyte cells specific for the autoimmune disease in a buffer and treating the suspended cells with a disrupting agent so as to produce activated T lymphocyte cells. Additionally, the resulting activating T lymphocyte cells may be treated with a chemical cross-linking agent.

The present invention also provides a composition for preventing or treating a specific autoimmune disease. This composition comprises pressure-treated, activated T lymphocyte cells of the present invention and a pharmaceutically acceptable carrier. Pharmaceutically acceptably carriers are known in the art and include, but are not limited to, 0.01-0.1M, preferably 0.05M, phosphate buffer, or 0.8% saline. Another composition for preventing or treating a specific autoimmune disease is also provided. This composition comprises chemical cross-linking agent-treated, activated T lymphocyte cells of the present invention and a pharmaceutically acceptable carrier. Yet a further composition for preventing or treating a specific autoimmune disease is provided by the present invention. This composition comprises disrupting agent-treated, activated lymphocyte cell membrane fragments of the present invention and a pharmaceutically acceptable carrier.

Appropriate routes of administration of the preparations of the present invention include oral, intranasal or transdermal administration, as well as intramuscular, intravenous, intradermal, subcutaneous and intraperitoneal injections.

The present invention further provides a method for recovering purified membrane proteins from activated T lymphocyte cells useful for preventing or treating a specific autoimmune disease. This method comprises initially suspending activated T lymphocyte cells specific for the autoimmune disease in a buffer. The suspended cells are subjected to a suitable hydrostatic pressure for an appropriate period of time so as to cause membrane proteins to be shed. Thereafter, the pressure on the suspended cells is released at an appropriate rate. The depressurized suspension is centrifuged to remove cells and produce a supernatant containing the membrane proteins. This supernatant may be ultracentrifuged so as to recover the purified membrane proteins. In one embodiment of the invention, the suspended cells, prior to being subjected to the suitable hydrostatic pressure, are treated with a chemical cross-linking agent. In another embodiment of the invention, the suspended cells, subsequent to being depressurized, are treated with a chemical cross-linking agent. In a further embodiment of the invention, the suspended cells, prior to being subjected to a suitable hydrostatic pressure, are treated with a disrupting agent. In yet a further embodiment of the invention, the depressurized cells may be treated with a disrupting agent. In still another embodiment of the invention, the suitable hydrostatic pressure is from 50.7 to 152 MPa (500 to 1500 atmospheres). In yet another embodiment of the invention, prior to ultracentrifuging the supernatant, the shed membrane proteins are treated with a chemical cross-linking agent.

The various cells, treatments and proteins provided by the present invention will be better understood by reference to the following experiments and examples which are provided for purposes of illustration and are not to be construed as in any way limiting the scope of the present invention, which is defined by the claims appended hereto.

Materials and Methods

6

Rats:

Inbred Lewis rats were obtained from the Animal Breeding Center of The Weizmann Institute of Science. Rats were used at 2-3 months of age and were matched for age and sex in each experiment.

Antigens:

Heat-killed M. tuberculosis $H_{37Ra}$ was purchased from Difco Laboratories (Detroit, MI). Purified protein derivative (PPD) of mycobacterium was purchased from Staten Serum Institute (Copenhagen, Denmark) and concanavalin A (Con A) from Bio-Yeda (Rehovot, Israel). Purified rat type II collagen was kindly donated by Dr. E.J. Miller of the University of Alabama Medical Center, Birmingham. Rat type I collagen was prepared from rat tail tendons and donated by Dr. D. Duskin of the Department of Biophysics of the Weizmann Institute (16).

Culture medium:

All cell cultures used Dulbecco's modified Eagle's medium (Grand island Biological Co., (GIBCO), Grand Island, New York). Media used for proliferation assays, for activation, and for cloning (proliferation medium) was supplemented with 1 mM glutamine (Bio-Lab, Jerusalem, Israel), 2-mercaptoethanol ($5x10^{-3}$M), gentamycin (40 micrograms/ml), and 1% fresh autologous rat serum. The medium used to maintain the propagated cell lines and clones in long-term culture (propagation medium) was the proliferation medium supplemented with 15% (vol/vol) of supernatant of ConA-activated lymphocytes as a source of T cell growth factor (3), 10% horse serum (GIBCO), 1 mM sodium pyruvate, and nonessential amino acids (Bio-Lab).

Introduction of active adjuvant athritis (AA)

To induce active AA, rats were inoculated intradermally at the base of the tail with 0.1 ml of complete Freund's adjuvant (CFA) containing 10 mg/ml MT in incomplete Freund's adjuvant (Difco Laboratories). The system described in (17) was used to assess severity of arthritis. Each paw was graded from 0 to 4 based on erythema, swlling, and deformity of the joint. The highest score achievable was 16. The clinical diagnosis of AA was confirmed by histologic examination of the joints of selected rats as in (18).

Maintenance and cloning of line A2

Line A2 reactive to MT was isolated from rats immunized with CFA. On day 9 the draining lymph nodes were removed, and a T cell line reactive to MT was generated and maintained as described (19). The limiting dilution technique was employed to clone A2. The line cells were activated in vitro by incubating them with MT in the presence of accessory cells, as described below, for 3 days. On the third day, the lymphoblasts were transferred into propagation medium or another 7 day period. On the 10th day, the cells were collected, washed, counted three times, and vigorously mixed. Cells were seeded at 0.1 cells/well in the presence of irradiated (1,500 rad) thymus cells ($2x10^7$/ml) and MT (10 micrograms/ml) into 96-well microtiter plates in 100 microliters of proliferation medium per well. Wells were checked for cell growth from the 6th to the 14th day after cloning. Plating efficiency was 60%. Cloned cells were expanded in propagation medium in 200 microliter wells and then in 2-, 6-, and finally, 10-ml plates at a concentration of $2-4x10^5$ cells/ml. Cultures were transferred every 3-4 days. Once every 2-4 weeks, the lines cells were reactivated by incubation with MT and accessory cells for 3 days (see below), and then transferred back into propagation medium. Cloned sub-line A2b was propagated in this manner for 6 weeks before its proliferative response to antigens or its ability to mediate AA or induce resistance was studied.

Activation of line cells

Cells ($2x10^5$/ml) of line A2 or subline A2b were activated by incubation for 3 days with 10 micrograms/ml of MT or 2.5 micrograms/ml of Con A in the presence of syngeneic irradiated (1,500 rad) thymus cells ($15x10^6$/ml) as accessory cells in the proliferation medium. After 3 days the lymphoblasts were collected, washed twice in phosphate-buffered saline (PBS), and were either transferred into propagation medium or used for in vivo or in vitro studies. By this time most of the irradiated thymus cells had died and disintegrated.

7

## Cell modifications

### A. Hydrostatic pressure

For pressure application on small volumes (2ml), the cell suspension was placed in a plastic capped tube and sample buffer was added to the top. After sealing, a short 22G needle was inserted through the cap to act as a vent for pressure equilibration. Air bubbles were avoided. The tube was placed in the cavity of a 45 ml pressure bomb (French Press Cell, Aminco). For larger sample volumes the cell suspensions were added directly into the cavity of the pressure bomb.

Hydrostatic pressure was gradually applied at a rate of 250 bar $min^{-1}$, maintained for up to two hours at the desired pressure and thereafter slowly released. The cells were collected and washed twice in PBS.

### B. Cross-linking of membrane proteins

Cells were fixed by formaldehyde or glutaraldehyde (0.3-1%) for 15 min. $10^6$-$10^8$ cells are suspended in 2.5 ml PBS. The same volume of a double concentrated solution of cross-linking agent in PBS was added to the cell suspensions at room temperature, after which cells were washed six times in PBS.

### C. Cytoskeletal disruption

The process was performed by the method described in "Cross-linking of membrane proteins", but a combination of cytochalasin (0.5 micrograms/ml) plus colchicine (10 micromolar), was used in place of the cross-linking agent.

### D. Membrane preparation

Fixed cell membranes were obtained by suspending cells in PBS hypotonic solution (1/3 physiological osmolarity + PMSF (10-5 M) + azide (0.02%)) for 15 min, 4°C in 5 ml vol., them homogenized in polytron homogenizer (2.5 min at intervals of 30 s, speed 6.5 units, at 4°C). The membranes were isolated using a discontinuous sucrose gradient (41% sucrose, 96,000 g, 2 h, Rotter SW28, 4°C). Separated membranes were dialyzed against PBS (2 hours, 4°C), and measured by Bradford analysis. The yield of proteins achieved by using this method was 1.5-2 mg per $3x10^8$ cells.

### EXPERIMENTAL MODELS:

Special in vitro lines of autoimmune T cells were developed (1-9). Table 1 summarizes three experimental autoimmune diseases associated with these lines of T lymphocytes. Experimental autoimmune encephalomyelitis (EAE), can be induced actively in genetically susceptible strains of rats by immunizing them to the basic protein of myelin (BP) (10). EAE is usually manifested as an acute disease characterized by paralysis and cellular infiltration in the white matter of the central nervous sytem. Untreated rats usually recover spontaneously from acute EAE after 2 or 3 days and are found to be resistant to further attempts to induce active EAE (2). Chronic or relapsing EAE can also be induced under certain conditions and such disease is similar in many respects to multiple sclerosis in man.

Experimental autoimmune thyroiditis (EAT) can be induced in H-2 genetically susceptible mice by immunizing thyroglobulin (Tg) in adjuvant (11). EAT is expressed as chronic inflammation of the thyroid gland. Strains of mice resistant to the lesion of EAT may yet produce high titers of Tg autoantibodies. EAT appears to be a model of autoimmune thyroiditis (Hashimoto's thyroiditis) that is not uncommon in humans.

Adjuvant arthritis (AA) differs from EAE and EAT in that it is induced in rats by immunizing them not to a defined self-antigen but to Mycobacterium tuberculosis organisms (Tb) in complete Freund's adjuvant (CFA) (12). About two weeks after inoculation, genetically susceptible rats develop a subacute polyarthritis with features reminiscent of some of those seen in rheumatoid arthritis in humans. It has been suggested that collagen type II might be the target self-antigen in AA, as arthritis may be induced by immunization to this antigen (13). However, recent evidence indicates that AA and collagen II arthritis may be separate entities (14, 15).

Table I also illustrates that similar autoimmune lesions may be induced by inoculation of antigen-specific line cells. The details of raising and maintaining the line cells and producing the diseases have been published (1-5). The basis of the method is to prime animals with the antigen of choice and select the specifically reactive cells by culture with the selecting antigen together with irradiated syngeneic accessory cells. The antigen-presenting accessory cells must be syngeneic, at least in part of the major histocompatibility complex (MHC), to trigger the proliferative response of line cells (3, 4). The selected line cells are then carried in culture with conditioned medium in the absence of antigen or accessory cells. Stable lines capable of mediating autoimmune diseases have all been found to be positive for general T cell markers (Thy 1 in mice or W3/13) and for the markers of delayed type hypersensitivity/helper cells (Lyt-1 or W3/25) with a few or no cells positive for the Lyt-2 or OX8 mark of cytotoxic/suppressor cells. None of the line cells are positive for Ig markers. To mediate disease the T lymphocyte line must be activated by incubation with a specific antigen or T cell mitogen before inoculation into recipient animals. A single inoculation of as few as $10^4$ -$10^5$ anti-BP or anti-Tg cells can lead to the clinical and pathological signs of marked EAE and EAT in a relatively short time. Production of AA requires the use of greater numbers of line cells ($10^7$) and relatively heavy irradiation of the recipient rate (750 R). Recipients must be syngeneic with the line cells at part of the MHC for disease to occur. The characteristic autoimmune lesions are accompanied by immunologically specific accumulation of line cells in the target organ. No evidence indicating a role for autoantibodies in disease produced by the T lymphocyte line cells is evident.

We have also succeeded in producing encephalomyelitis or arthritis cloned cells; the anti-BP clones have been somewhat less virulent than their parent lines while an anti-Tb clone has been isolated that is much more virulent than its parent.

Vaccination against autoimmune disease

The use of line cells as specific vaccines to induce resistance to autoimmune disease is summarized in Table II. Anti-BP line cells subjected to irradiation or treated with mitomycen C were no longer capable of producing EAE. However, a single intravenous inoculation of such attenuated line cells led to resistance in about 65% of rats induced actively by immunization with BP/CFA. In early experiments the rats were still susceptible to EAE produced by passive transfer of anti-BP line cells, suggesting that the mechanism of resistance might be less effective against preformed effect or cells than against differentiating cells (7). However, we have recently observed that it is possible to prevent EAE due to positive transfer of line cells as well as active EAE using pressure-treated cells (20). In contrast, a single intravenous inoculation of attenuated anti-Tg line cells was found not only to completely inhibit active EAT induced by Tg/CFA, but also to prevent EAT mediated by inoculation of activated anti-Tg line cells. Thus, in principle resistance to autoimmune disease is not limited to the early phases of differentiation but can include the effector phase of disease. See Table V for results of vaccination experiments performed with pressure, cross-linking agent or disrupting-agent treated T lymphocytes. Furthermore, see Figures 3-5 for results of vaccination experiments involving T lymphocytes modified by various methods disclosed by the present invention.

Therapy with membrane proteins

Autoimmune line cells were found to be effective as agents to prevent and treat experimental autoimmunity. This approach may help in the management of clinical autoimmune diseases, illnesses for which their exists no specific mode of therapy. Although the clinical emphasis must be on treatment rather than on prevention, it is possible that in practice this distinction will not be critical. Autoimmune diseases of serious concern are often progressive or relapsing and prevention of the differentiation of fresh waves of autoimmune lymphocytes may, by itself, constitute effective therapy.

Fig. 1. illustrates alleviation of AA by a single inoculation of line cells. In this experiment groups of rats suffering from actively induced AA were treated with specific anti-Tb line cells or with control line cells. The rats treated with the specific line cells had less severe disease and a hastened remission.

Another consideration is the identification and availability of self-antigens to which the autoimmune lymphocyte lines should be selected. In many conditions the self-antigens are unknown or may be in very limited supply. Nevertheless, the AA model suggests that it ought to be possible to raise relevant cell lines using mixtures of poorly defined antigens obtained even from foreign sources. Why or how specifically virulent autoimmune cells should emerge under such conditions is puzzling, but a fact. However, it is not necessary to raise lines or clones of T lymphocytes to obtain effective vaccines. Lymph node cells taken directly from rats or mice with AA, EAE, or EAT can be activated with a specific antigen or a T lymphocyte mitogen, and treated with pressure, cross-linking agents, or disrupting-agents, and the cells used to obtain the results described in Tables 4, 5 and 6.

It may be advantageous to effect therapy with subcellular material from line cells or with cells of augmented antigenicity and its has been found that membrane proteins may be used effectively. Membrane proteins of line cells were prepared by a novel method which was previously applied for isolation of blood group antigens from human erythrocytes. The method is based on the hypothesis that the equilibrium position of membrane proteins is displaced towards the aqueous domain upon rigidization of the membrane lipid bilayer, and at extreme lipid viscosities proteins are shed. In principle, each integral membrane protein has a defined threshold of lipid viscosity where is shed from the membrane.

The most efficient manner of hyper-rigidization of membranes is by the application of hydrostatic pressure 50.7 to 152 MPa (500 to 1500 atm) which can be augmented by pretreatment with cholesterol. Cells, in general, survive such treatment and the material which is shed can be fractionated according to size by centrifugation. Material which remains in the supernatant after centrifugation at 100,000 g for 1 hour can be considered as isolated proteins or small aggregates thereof. The precipitate of this centrifugation consists of membrane fragments and large protein aggregates. The soluble membrane proteins retain in general their activity, in contrast to membrane proteins isolated by the conventional use of detergents.

The capacity of immunization against autoimmune diseases was found in the following fractions: a) pressurized activated cells (presumably due to lateral rearrangement and vertical displacement of the specific antigen receptors; b) the shed soluble proteins; and c) the membrane fragments.

Table III shows that membrane fractions isolated by the pressure method were immunologically specific in inhibiting the reaction of autoimmune line cells to their particular antigen. For example, the membrane fraction obtained from the Z1a anti-BP line inhibited the response of intact Z1a line cells to BP; it did not inhibit the response of arthritis-producing A2 line cells to their antigen. Conversely, the membrane fraction obtained from arthritis producing A2 cells inhibited the response of intact A2 line cells, but not of Z1a line cells. These results indicate that the membranes contain biologically active receptors specific for self antigens.

Figure 2 shows the results of an experiment in which rats were administered two doses each of 0.05 micrograms of a membrane fraction of Z1a line cells at weekly intervals, and two weeks later active EAE was induced in the rats. It can be seen that the rats treated with the membrane fraction suffered very mild paralysis compared to the control rats. Thus, the course of the disease could be markedly alleviated using specific membrane factions. Table IV shows the results of vaccinating rats with pressure activated anti-BP line cells. It can be seen that rats treated with control line cells were susceptible to EAE while rats treated with pressure activated, specific anti-BP line cells were completely resistant to active EAE. They were also resistant to EAE mediated by intact, anti-BP line cells (not shown). See also Table VI for results of pressure-treated, cross-linking agent treated membrane preparations used as a vaccine against AA.

Examples of autoimmune diseases that can be treated using membrane protein of autoimmune line cells:

| Human autoimmune diseases | Antigen used to activate T lymphocytes | |
|---|---|---|
| Multiple sclerosis | a) | Myelin basic protein |
| | b) | Crude extract of central nervous system |
| Thyroiditis | a) | Thyroglobulin |
| | b) | Crude extract of thyroid gland |
| Diabetes (Type I) | a) | Extract of Islet cells |
| Ankylosing spondylitis (specific types) | a) | Certain Klebsiella bacteria |
| | b) | Crude extract of joints |
| Rheumatoid arthritis | a) | Crude extract of joints |

EXAMPLE 1

Preparation of Vaccine

Cells from a line of T lymphocytes directed against the myelin basic protein were used (Z1a line). These cells induce experimental autoimmune encephalitis (EAE) in rats. $6 \times 10^8$ cells, suspended in 2.5 ml of phosphate buffered saline pH 7.2 (PBS), were placed in a sterile pressure chamber. The lid was placed directly on top of the solution to eliminate any air bubbles. Pressure was applied gradually by a French Press (15 min) to reach 1000 X 0.1013 MPa (1000 atmospheres) and maintained at this pressure for 45 min. The pressure was then slowly reduced (15 min) to atmospheric pressure. The cells were transferred to a test tube, spun down at 1500 rpm and the precipitate was separated. Cell viability, tested by trypan blue exclusion, was over 90%. The supernatant was then ultracentrifuged at 100,000 g for one hour. The supernatant (about 600 micrograms proteins in 2 ml PBS) was collected and used in the in vitro and in vivo functional experiments described below.

In the in vitro experiment, cells of the Z1a line were induced to proliferate by the addition of their specific antigen (0.2 micrograms/ml myelin basic protein).

Cell proliferation was expressed by counts per minute of incorporated radioactive thymidine. The addition of 30 micrograms/ml of the shed proteins (see above) resulted in reduction of cell proliferation by 40-60%. When 30 micrograms/ml of proteins shed from other T line cells (including arthritis) were used, no effect on cell proliferation was observed. Typical results of such an experiment are shown in table III, and indicate that the material shed from the cell surface pressure includes a specific receptor to the inducing antigen. This material may be used for immunization against the receptor resulting in partial or complete elimination of the autoimmune disease. Such an experiment is described below.

Rats were first preimmunized twice at a week's interval by inoculation of 0.5 micrograms soluble proteins shed by pressure of the Z1a line cells. Two weeks later the rats were challenged with BP antigen in adjuvant. After 14 days all rats in the control group manifested severe EAE while the pretreated rats

11

showed only a mild form of EAE. Typical profiles after challenge with an encephalitogenic dose of BP are shown in Fig. 2. Again, pretreatment of rats with proteins shed by pressure of an unrelated line (arthritis) showed no effect on the development of EAE.

TABLE I

Experimental Autoimmune Diseases Actively Induced, or Produced by Autoimmune T Lymphocyte Line Cells

| | | | Actively Induced disease | | | | Disease produced by line cells | | | | |
|---|---|---|---|---|---|---|---|---|---|---|---|
| Disease | Species | Target Organ | Self-Antigen | Immunization | Latency (days) | Course | Antigen for line selection | Cell Inoculum | Recipients | Latency (days) | Course |
| EAE* | Rat | White matter of CNS | BP | BP/CFA | 12 | Acute | BP | $10^4$-$10^5$ | Intact | 3-6 | Acute |
| EAT | Mouse | Thyroid | Tg | Tg/CFA | 30 | Chronic | Tg | $10^4$-$10^5$ | Intact | 1-3 | Chronic |
| AA | Rat | Joints | ? | CFA | 14 | Subacute | Tb | $10^7$ | Irradi-ated | 5-10 | Subacute |

*Abbreviations used in Tables :

AA, adjuvant arthritis; BP, myelin basic protein; CFA, complete Freund's adjuvant; CNS, central nervous system;

EAE, experimental autoimmune encephalomyelitis; EAT, experimental autoimmune thyroiditis; IFA, incomplete Freund's

adjuvant; Tb, Mycobacterium tuberculosis organisms; Tg, thyroglobulin.

### Table II
### Vaccination Against Autoimmune Diseases Using Specific
### Autoimmune Line Cells

| Disease | Vaccine | | Resistance to Disease | | |
|---------|---------|-----------|---------|------|--------|
| | Line cells | Treatment | Activity induced | Line mediated | Degree o resistan |
| EAE | anti-BP | Irradiation | yes | no | 60-70% |
| EAT | anti-Tg | Irradiation | yes | yes | complet autoant bodies develop complet |
| AA | anti-Tb | none | yes | ? | complet |

Rats or mice inoculated intravenously with activated line cells (anti-BP, $5 \times 10^6$; anti-Tg, $5 \times 10^6$; anti-Tb, $2 \times 10^7$), some of which had been treated by irradiation (1,500 R). Control animals (not shown) were inoculated with line cells directed against irrelevant antigens. Two to four weeks later, the animals were challenged to induce active autoimmune diseases.

### Table III
### Membrane fractions inhibit proliferative responses of
### specific line cells

| Cellular orgin of membrane fraction | Proliferative response to specific antigen | |
|---|---|---|
| | % inhibition of line | |
| | A2 | Z1a |
| A2 | 39 | 0 |
| A1a | 0 | 50 |

Membrane fractions from the A2 (arthritis) and the Z1a (encephalomyelitis) cell lines were obtained using the pressure method (1,000 atm) and 50 micrograms/ml were included in the proliferative responses to specific antigen of the intact line cells. The % inhibition was computed by comparing the response in the presence of the membrane fraction obtained from the specific line cells to the response in the presence of the membrane fraction for the other line cells.

Experiments carried out with pressure-treated, activated cells gave practically identical results.

Table IV

Vaccination against EAE using pressure-treated,
activated anti-BP line cells

| Treatment of rats with pressure-treated activated line cell | EAE induced by active immunization with BP/CFA | |
|---|---|---|
| | Incidence | Clinical severity |
| Control | 30/30 | moderate to severe |
| Anti-BP | 5/30 | none to very mild |

Lewis rats were inoculated intraperitoneally with anti-BP or control line cells ($5 \times 10^6$ weekly x4) that had been activated and treated with hydrostatic pressure (1150 atm. for 15 min.) and challenged 1 week later with BP/CFA to induce active EAE.

Table V

Vaccination against experimental autoimmune
thyroiditis using T lymphocyte vaccines

| T Lymphocyte Vaccine | Treatment of T lymphocytes | Percent Indicidence of Thyroiditis |
|---|---|---|
| None | None | 100 |
| Anti-Tg | Pressure | 45 |
| Anti-Tg | Colchicine + cytochalasin | 59 |
| Anti-Tg | Formaldehyde | 35 |

Groups of 10 mice each of strain C3H/eBxC57BL/6J)F1 were induced to develope experimental autoimmune thyroiditis by an intravenous inoculation of $2 \times 10^6$ anti-thyroglobulin (anti-Tg) line T lymphocytes. Before induction of disease, some mice were treated with 3 weekly subcutaneous injections of $10 \times 10^6$ activated anti-Tg line T cells that had been treated with pressure, colchicine plus cytochalasin, or formaldehyde, as described in the legend to Figure 3. The severity of thyroiditis was graded by histological examination of the thyroid glands 1 week later.

14

Table VI

Vaccination against AA using isolated membrane
preparations of pressurized and cross-linked T lymphocytes

| Vaccination with A2b clone membranes | | Induction of AA by MT | |
|---|---|---|---|
| Pressure | Formaldehyde | Incidence of AA | Mean AA score on day 20 |
| None | None | 100 | 53 |
| Yes | None | 100 | 55 |
| Yes | Yes | 50 | 15 |

Groups of 10 Lewis rats were or were not vaccinated subcutaneously 4 times at weekly intervals with 1.2 mg of A2b isolated membrane preparations. One week later the rats were challenged with MT (1 mg in oil) to induce AA. The clinical score on day 20 of affected rats is recorded at a scale of 0 to 100% as described in the legend to Figure 3.

Isolated membranes were prepared as follows: $3 \times 10^8$ activated A2b T cells were treated with hydrostatic pressure (1500 bars for 15 min.) and one half of the cells were also treated with the cross-linker formaldehyde (0.3%, 15 min.) and washed six times in PBS. Membranes were obtained by lysing the cells in a hypotonic solution of NaCl (1/3 physiological osmolarity + PMSF ($10^{-5}$ M) +azide (0.02%). The cells were homogenized in a polytron homogenizer (6.5 min., 2.5 units for 30 second at 4°C) and the membranes isolated with a 41% sucrose gradient (96,000g, 2 h, Rotter SW28, at 4°C). The membranes were dialyzed against PBS (2 h, 4°C) and proteins were measured by Bradford analysis: The yield of membrane protein was 1.5-2mg.

EXAMPLE 2

Prevention of autoimmune diabetes mellitus using treated lymphocytes from diabetic mice

C57BL/KS mice develop autoimmune diabetes upon exposure to 5 daily doses (50 mg/kg each) of the toxin streptozotocine (STZ) (21). At this dose STZ induces diabetes by causing mild damage to the pancreatic beta cells which normally produce insulin. As a result the T lymphocytes of the STZ exposed mice recognize and attack certain undefined beta cell antigens leading to autoimmune destruction of the animals' beta cells clinical diabetes.

Experiments were performed using spleen T lymphocytes of STZ-exposed mice to vaccinate against diabetes. The spleen lymphocytes were incubated for two days with the T cell mitogen concanavalin A to activate the autoimmune T cells present in the spleen responsible for attacking the beta cells. These activated lymphocytes were then treated by incubation for 15 minutes with the chemical cross-linker glutaraldehyde at a concentration of 0.3% in phosphate buffered saline. The glutaraldehyde-treated T cells were washed six times in fresh phosphate buffered saline and groups of 20 C57BL/KS mice were inoculated subcutaneously with $2 \times 10^7$ activated glutaraldehyde-treated T cells. Two weeks later the mice were exposed to a 5 day course of STZ to induce diabetes. The results are shown in Table VII.

15

<u>Table VII</u>

Prevention of autoimmune diabetes

by vaccination using glutaraldehyde-treated T cells

| T cell<br>vaccination[a] | Incidence pf<br>diabetes[b] |
|---|---|
| none | 100% |
| yes | 40% |

[a] Groups of 20 C57BL/KS mice were vaccinated or not with a single injection of $2 \times 10^7$ glutaraldehyde (0.3%)-treated, activated T cells obtained from the spleens of STZ-exposed diabetic mice.

[b] The mice were challenged with a course of STZ (50 mg/kg, daily for 5 days) and studied for the appearance of diabetes measured by glycosuria, hyperglycemia (>500 mg), and histological evidence of insulitis.

The relative resistance of the vaccinated mice to autoimmune diabetes indicates: a) the effectiveness of glutaraldehyde-treated T cell vaccines; b) the prevention of diabetes; and c)the ability to vaccinate without recourse to purified lines and defined antigens, using mitogen-activated spleen cells obtained directly from diabetic mice.

Accordingly, similar populations of T cells may be obtained from the blood of diabetic or other patients and used to prepare T cell vaccines.

EXAMPLE 3

T cell vaccines treated with hydrostatic pressure followed by glutaraldehyde specific vaccines

Groups of 20 Lewis rats were vaccinated at weekly intervals by three subcutaneous injections of $10^7$ activated T cell vaccines. The T cell vaccines were either an anti-BP or an anti-MT clone treated with hydrostatic pressure (1250 bars for 15 min) followed by gluteraldehyde (0.3% for 15 min). One week after the last vaccination the rats were challenged with BP (myelin basic protein; 25 micrograms) in adjuvant to induce EAE (experimental autoimmune encephalomyelitis) or with MT (Mycobacterium tuberculosis; 1 mg) in oil to induce AA (adjuvant arthritis).

The data in Table VIII below demonstrates that clones of activated T cells may be treated by a combination of pressure and cross-linking agents and used as specific vaccines.

16

## Table VIII

## Specific vaccination using activated T cells treated with hydrostatic pressure and gluteraldehyde

| T cell vaccine | Induced disease | Incidence | Severity |
|---|---|---|---|
| None | EAE | 100% | Lethal |
|  | AA | 100% | Severe |
| Anti-BP | EAE | 20% | Mild |
|  | AA | 100% | Severe |
| Anti-MT | EAE | 100% | Lethal |
|  | AA | 0 | - |

The results indicate: a) the effectiveness of a combination of hydrostatic pressure and a chemical cross-linker in preparing T cell vaccines; b) the immunological specificity of the vaccines (each vaccine induced resistance to the disease with which it was immunologically associated); and c)T cell vaccination leads to inhibition of T cell response to antigen.

In another experiment Lewis rats were vaccinated or not with anti-MT clone cells treated either by gamma irradiation or a combination of hydrostatic pressure and a chemical cross-linker. Specifically, groups of 10 Lewis rats were not vaccinated or were vaccinated by 3 weekly inoculations of $10^7$ A2b (anti-MT) clone cells that had been either irradiated (1500R) or treated with hydrostatic pressure (1250 bars for 15 min) followed by gluteraldehyde (0.3% for 15 min). The rats were then imunized 1 week later with MT (1 mg) in oil in the hind foot pads. The draining lymph nodes were removed 9 days later and their proliferative responses to MT in vitro were measured by the incorporation of $^3$H-thymidine into DNA. The results are shown in Table IX below.

## Table IX

## T cell vaccination leads to suppression of T cell reactivity to antigen

### Vaccination

| Anti-MT clone | Treatment of clone | Response to to MT (cpm) |
|---|---|---|
| None | None | 31,586 ± 200 |
| A2b | Irradiation | 27,762 ± 300 |
| A2b | Pressure, gluteraldehyde | 6,500 ± 150 |

The results indicate the effectiveness of T cell vaccination with cells treated by a combination of pressure and gluteraldehyde in suppressing T cell reactivity to an antigen.

EXAMPLE 4

Therapeutic vaccination of established adjuvant arthritis using treated cells obtained from arthritic animals

17

The clinical use of T cell vaccination against human autoimmune disease may be used: 1) for the treatment of individuals who are already suffering from a particular autoimmune disease, rather than for prevention of future disease; 2) as a vaccine of autoimmune T cells obtained from the sick individual rather than from a healthy individual; and 3) for the preparation of vaccines from populations of cells removed directly from the individual, rather than preparation of vaccines from long term cell lines or clones.

The following experiments show the satisfaction of these conditions in the model of adjuvant arthritis.

Adjuvant arthritis was induced in a group of 30 Lewis rats by immunizing each rat with 1 mg of Mycobacterium tuberculosis organism (H37Ra) in oil. On day 15, when all the rats were suffering from overt arthritis, 3 groups of 10 rats each were subcutaneously vaccinated or not with lymph node cells ($50 \times 10^6$) obtained from another group of rats which was also suffering from severe adjuvant arthritis. The donor lymph node cells were activated by incubation with the T cell mitogen concanavalin A (1.5 micrograms/ml for 48 h). Some of the donor cells were treated by irradiation (2500 R) alone, and some of the donor cells were treated with the chemical cross-linker glutaraldehyde (0.3%) for 15 min. and then extensively washed in saline. A control group of 10 rats was left untreated.

On day 17, the groups of rats received a booster vaccination with $50 \times 10^6$ donor cells treated with irradiation or glutaraldehyde as before. By day 24, all the rats vaccinated with the glutaraldehyde-treated donor cells were in complete lasting remission, while the untreated control rats and the rats treated with irradiated donor cells continued to suffer from ongoing adjuvant arthritis. Table X below summarizes the results.

## Table X

### Therapeutic vaccination against adjuvant arthritis

| Donor node cells from arthritic rats ($50 \times 10^6$) | Treatment of lymph node cell vaccine | Adjuvant arthritis on day 25 and onward | |
|---|---|---|---|
| | | Incidence | Severity |
| none | none | 10/10 | severe |
| yes | irradiation (2500 R) | 10/10 | moderate-severe |
| yes | gluaraldehyde (0.3%) | 0/10 | none |

These results indicate that : 1) vaccines may be prepared directly from ill individuals; 2) glutaraldehyde (chemical cross-linker) treatment of cells renders them an effective vaccine, whereas irradiation does not; and 3) treatment of established disease is attainable.

EXAMPLE 5

T cell vaccination against delayed type hypersensitivity (DTH) to a skin sensitizing antigen

In addition to autoimmune diseases, T-lymphocytes mediate delayed type hypersensitivity (DTH) reactions to foreign chemicals such as oxazolone (DX) or dinitrofluorobenzene (DNFB). To demonstrate the general applicability of T cell vaccination by showing its effect on inhibiting DTH, groups of 10 BALB/c mice were sensitized to OX or DNFB by painting the skin of the mice with the particular chemical in a suitable vehicle. DTH allergy to the chemical was tested 5 days later by applying the chemical to the ears of the mice and measuring the increase in ear thickness 24 h later. Mice were vaccinated by subcutaneous injection of $20 \times 10^7$ lymph node cells from donor mice that had been sensitized 5 days earlier to the chemical. The donor lymph node cells were activated by incubation with concanavalin A for 48 hr and were then treated either by irradiation (2500 R) or by glutaraldehyde (0.3%) for 15 min before using them as vaccines. Typical results are shown in Table XI below.

## Table XI

### Vaccination of mice against DTH to OX.

| Vaccine | | DTH in test mouse | |
|---|---|---|---|
| Sensitization of donor mice | Treatment of donor lymph node cell vaccine | Sensitization antigen | % inhibition of DTH |
| none | none | OX | 0 |
| none | none | DNFB | 0 |
| OX | irradiation | OX | 0 |
| OX | glutaraldehyde | OX | 60 |
| OX | glutaraldehyde | DNFB | 0 |

The data in Table XI demonstrate that lymph node cells of donor mice sensitized to OX may be used vaccinate test mice against DTH reactivity to OX, but not against DTH reactivity to DNFB, provided that the donor vaccine was composed of glutaraldehyde-treated cells. Irradiated donor cells did not vaccinate.

These results indicate that:

1) Chemically cross-linked activated lymph node cells vaccinate in an immunologically specific manner against DTH reactivity;

2) Irradiated donor cells do not vaccinate;

3) Vaccination with cells is effective against foreign antigens as well as against self-antigens and autoimmunity;

4) Donor cells need not be activated with the specific antigen to serve as vaccine, but can be activated with a mitogen such as concanavalin A; and

5) Lymph node cells, as well as T cell lines and clones, may be used effectively.

REFERENCES

1. Ben-nun, A., Wekerle, H. and Cohen, I.R. (1981). Eur. J. Immun. 11: 195-199.

2. Ben-nun, A. and Cohen, I.R. (1982). J. Immunol. 128:1450-1457.

3. Ben-Nun, A. and Cohen, I.R. (1982). J. Immunol. 129:303-308.

4. Ben-Nun, A., Eisenstein, S. and Cohen, I.R. (1982). J. Immunol. 129:918.

5. Holoshitz, J., Naparstek, Y., Ben-Nun, A., and Cohen, I.R. (1983). Science 219:56-58.

6. Ben-Nun, A., Wekerle, H. and Cohen, I.R. (1981). Nature 292:60-61.

7. Ben-Nun, A. and Cohen, I.R. (1981). Eur. J. Immunol. 11:949-952.

8. Maron, R., Zeubavel, R., Friedman, A. and Cohen, I.R. (1983). J. Immunol. 131:2316-2320.

9. Holoshitz, J., Frenkel. A., Ben-Nun, A. and Cohen, I.R. (1983). J. Immunol. 131:2810-2813.

10. Paterson, P.Y. (1976) In: Textbook of Immunopathology. (Meischer, P. and Muller-Eberhard, H.J. Eds) 2nd Ed., pp. 179-213. Grune & Stratton, N.Y.

11. Rose, N.R., Twarog, F.J. and Crowle, A.J. (1971). J. Immunol. 106:698-708.

12. Pearson, C.M. (1963). J. Chronic Dis. 16:863-874.

13. Trentham, D.E. McCunr, W.J. Susman, P. and David, J.R. (1980). J. Clin. Invest. 66:1109-1117.

14. Iizuka, Y. and Chang, Y.H. (1982). Arthritic Rheum. 25:1325-1332.

15. Holoshitz, J., Matitiau, A. and Cohen, I.R. (1984). J. Clin. Invest. 73:211-215.

16. Duskin, et al., (1985). Cell 5:83-86.

17. Trentham, D.E., et al. (1977). J. Exp. Med. 146:857.

18. Pearson, C.M. (1956). Proc. Soc. Exp. Bio. Med. 91:95.

19. Holoshitz, J., et al., (1983). Science 219-56.

20. Lieder, O., et al., (1986. Ann. N.Y. Acad. Sci. 475: 267.

21. Like, A.A. and Rossini, A.A. Science 193:415-417 (1986).

**Claims**

# EP 0 261 648 B1

1. A pressure-treated, activated T lymphocyte cell useful for preventing or treating a specific autoimmune disease, which has been pressure treated by means of hydrostatic pressure in the order of 50.7 to 152 MPa (500 to 1500 atm) for sufficient time to cause augmented antigenicity of the T lymphocyte cells without substantial loss of membrane proteins from the cells and which additionally has been treated with a chemical cross-linking agent.

2. A pressure-treated, activated T lymphocyte cell of claim 1, wherein the specific autoimmune disease is multiple sclerosis, thyroiditis, diabetes type I, ankylosing spondylitis, rheumatoid arthritis, or myasthenia gravis.

3. A pressure-treated, activated T lymphocyte cell of claim 1 which has been treated with the chemical cross-linking agent after being treated with pressure.

4. A pressure-treated, activated T lymphocyte cell of claim 1 which has been treated with the chemical cross-linking agent prior to being treated with pressure.

5. A pressure-treated, activated T lymphocyte cell useful for preventing or treating a specific autoimmune disease, which has been pressure treated by means of hydrostatic pressure in the order of 50.7 to 152 MPa (500 to 1500 atm) for sufficient time to cause augmented antigenicity of the T lymphocyte cells without substantial loss of membrane proteins from the cells and which additionally has been treated with a cytoskeletal disrupting agent.

6. A pressure-treated, activated T lymphocyte cell of claim 5 which has been treated with the disrupting agent after being treated with pressure.

7. A pressure-treated, activated T lymphocyte cell of claim 5 which has been treated with the disrupting agent prior to being treated with pressure.

8. A chemical cross-linking agent-treated, activated T lymphocyte cell useful for preventing or treating a specific autoimmune disease.

9. A chemical cross-linking agent-treated, activated T lymphocyte cell of claim 8, wherein the specific autoimmune disease is multiple sclerosis, thyroiditis, diabetes type I, ankylosing spondylitis, rheumatoid arthritis, or myasthenia gravis.

10. A disrupting agent-treated, activated T lymphocyte cell useful for preventing or treating a specific autoimmune disease.

11. A disrupting agent-treated, activated T lymphocyte cell of claim 10, wherein the specific autoimmune disease is multiple sclerosis, thyroiditis, diabetes type I, ankylosing spondylitis, rheumatoid arthritis, or myasthenia gravis.

12. A disrupting agent-treated, activated T lymphocyte cell of claim 10 which has additionally been treated with a chemical cross-linking agent.

13. A method for producing pressure-treated, activated T lymphocytes useful for preventing or treating a specific autoimmune disease according to claim 1 which comprises:
    a) suspending activated T lymphocyte cells specific for the autoimmune disease in a buffer;
    b) subjecting the suspended cells to a chemical cross-linking agent;
    c) subjecting the treated cells to a suitable hydrostatic pressure in the order of 50.7 to 152 MPa (500 to 1500 atm) for sufficient time to cause augmented antigenicity of the T lymphocyte cells without substantial loss of membrane proteins from the cells; and
    d) releasing the pressure on the suspended cells at an appropriate rate so as to produce Pressure-treated, activated T lymphocyte cells without substantial loss of membrane proteins from the cells.

14. A method according to claim 12, wherein the cells are additionally treated with a disrupting agent.

15. A method for producing pressure-treated, activated T lymphocytes useful for preventing or treating a

20

specific autoimmune disease according to claim 1 which comprises:

a) suspending activated T lymphocyte cells specific for the autoimmune disease in a buffer;

b) subjecting the suspended cells to a suitable hydrostatic pressure in the order of 50.7 to 152 MPa (500 to 1500 atm) for sufficient time to cause augmented antigenicity of the T-lymphocyte cells without substantial loss of membrane proteins from the cells;

c) releasing the pressure on the suspended cells at an appropriate rate so as to produce Pressure-treated, activated T lymphocyte cells without substantial loss of membrane proteins from the cells; and

d) subjecting the pressure treated cells to a chemical cross-linking agent.

16. A method according to claim 15, wherein the cells are additionally treated with a disrupting agent.

17. A method for producing pressure-treated, activated T lymphocytes useful for preventing or treating a specific autoimmune disease according to claim 5 which comprises:

a) suspending activated T lymphocyte cells specific for the autoimmune disease in a buffer;

b) subjecting the suspended cells to a cytoskeletal disrupting agent;

c) subjecting the treated cells to a suitable hydrostatic pressure in the order of 50.7 to 152 MPa (500 to 1500 atm) for sufficient time to cause augmented antigenicity of the T lymphocyte cells without substantial loss of membrane proteins from the cells; and

d) releasing the pressure on the suspended cells at an appropriate rate so as to produce pressure-treated, activated T lymphocyte cells without substantial loss of membrane proteins from the cells.

18. A method for producing pressure-treated, activated T lymphocytes useful for preventing or treating a specific autoimmune disease according to claim 5 which comprises:

a) suspending activated T lymphocyte cells specific for the autoimmune disease in a buffer;

b) subjecting the suspended cells to a suitable hydrostatic pressure in the order of 50.7 to 152 MPa (500 to 1500 atm) for sufficient time to cause augmented antigenicity of the T lymphocyte cells without substantial loss of membrane proteins from the cells;

c) releasing the pressure on the suspended cells at an appropriate rate so as to produce pressure-treated, activated T lymphocyte cells without substantial loss of membrane proteins from the cells; and

d) subjecting the pressure treated cells to a cytoskeletal disrupting agent.

19. A method for producing chemical cross-linking agent-treated, activated T lymphocytes useful for preventing or treating a specific autoimmune disease which comprises:

a) suspending activated T lymphocyte cells specific for the autoimmune disease in a buffer; and

b) treating the suspended cells with a chemical cross-linking agent.

20. A method for producing disrupting agent-treated, activated T lymphocyte cells useful for preventing or treating a specific autoimmune disease which comprises:

a) suspending activated T lymphocyte cells specific for the autoimmune disease in a buffer; and

b) treating the suspended cells with a disrupting agent so as to produce activated T lymphocyte cells having dissociated cytoskeletons.

21. A method according to claim 20, wherein subsequent to step b) the activated T lymphocyte cells having dissociated cytoskeletons are treated with a chemical cross-linking agent.

22. A composition for preventing or treating a specific autoimmune disease which comprises pressure-treated, cross-linking agent treated, activated T lymphocyte cells of claim 1 and a pharmaceutically acceptable carrier.

23. A composition for preventing or treating a specific autoimmune disease which comprises chemical cross-linking agent-treated, activated T lymphocyte cells of claim 8 and a pharmaceutically acceptable carrier.

24. A composition for preventing or treating a specific autoimmune disease which comprises disrupting agent-treated, activated T lymphocyte cell membrane fragments of claim 10 and a pharmaceutically acceptable carrier.

21

**25.** A method for recovering purified membrane proteins from activated T lymphocyte cells useful for preventing or treating a specific autoimmune disease which comprises:

a) suspending activated T lymphocyte cells specific for the autoimmune disease in a buffer;

b) subjecting the suspended cells to a chemical cross-linking agent;

c) subjecting the treated cells to a suitable hydrostatic pressure in the order of 50.7 to 152 MPa (500 to 1500 atm) for sufficient pressure and time to cause augmented antigenicity of the T lymphocyte cells without substantial loss of membrane proteins from the cells;

d) releasing the pressure on the suspended cells at an appropriate rate;

e) centrifuging the depressurized suspension to remove cell fragments and produce a supernatant containing the membrane proteins; and

f) treating the supernatant by ultracentrifugation so as to recover the purified membrane proteins.

**26.** A method for recovering purified membrane proteins from activated T lymphocyte cells useful for preventing or treating a specific autoimmune disease which comprises:

a) suspending activated T lymphocyte cells specific for the autoimmune disease in a buffer;

b) subjecting the suspended cells to a suitable hydrostatic pressure in the order of 50.7 to 152 MPa (500 to 1500 atm) for sufficient pressure and time to cause augmented antigenicity of the T lymphocyte cells without substantial loss of membrane proteins from the cells;

c) releasing the pressure on the suspended cells at an appropriate rate;

d) subjecting the pressure-treated cells to a chemical cross-linking agent;

e) centrifuging the depressurized suspension to remove cell fragments and produce a supernatant containing the membrane proteins; and

f) treating the supernatant by ultracentrifugation so as to recover the purified membrane proteins.

**27.** A method for recovering purified membrane proteins from activated T lymphocyte cells useful for preventing or treating a specific autoimmune disease which comprises:

a) suspending activated T lymphocyte cells specific for the autoimmune disease in a buffer;

b) subjecting the suspended cells to a cytoskeletal disrupting agent;

c) subjecting the treated cells to a suitable hydrostatic pressure in the order of 50.7 to 152 MPa (500 to 1500 atm) for sufficient pressure and time to cause augmented antigenicity of the T lymphocyte cells without substantial loss of membrane proteins from the cells

d) releasing the pressure on the suspended cells at an appropriate rate;

e) centrifuging the depressurized suspension to remove cell fragments and produce a supernatant containing the membrane proteins; and

f) treating the supernatant by ultracentrifugation so as to recover the purified membrane proteins.

**28.** A method for recovering purified membrane proteins from activated T lymphocyte cells useful for preventing or treating a specific autoimmune disease which comprises:

a) suspending activated T lymphocyte cells specific for the autoimmune disease in a buffer;

b) subjecting the suspended cells to a suitable hydrostatic pressure of sufficient pressure and time to cause augmented antigenicity of the T lymphocyte cells without substantial loss of membrane proteins from the cells;

c) releasing the pressure on the suspended cells at an appropriate rate;

d) subjecting the pressure-treated cells to a cytoskeletal disrupting agent;

e) centrifuging the depressurized suspension to remove cell fragments and produce a supernatant containing the membrane proteins; and

f) treating the supernatant by ultracentrifugation so as to recover the purified membrane proteins.

**29.** A method for recovering purified membrane proteins from activated T lymphocyte cells useful for preventing or treating a specific autoimmune disease which comprises:

a) suspending activated T lymphocyte cells specific for the autoimmune disease in a buffer;

b) treating the supernatant by ultracentrifugation so as to recover the purified membrane proteins;

c) subjecting the suspended cells to a suitable hydrostatic pressure of sufficient pressure and time to cause augmented antigenicity of the T lymphocyte cells without substantial loss of membrane proteins from the cells;

d) releasing the pressure on the suspended cells at an appropriate rate;

e) centrifuging the depressurized suspension to remove cell fragments and produce a supernatant containing the membrane proteins; and

f) subjecting the shed membrane proteins to a chemical cross-linking agent.

30. A preparation for preventing or treating an autoimmune disease, comprising T-lymphocyte cells which have developed specificity for the autoimmune disease specific antigen and which cells have been activated either by incubating in the presence of the autoimmune disease specific antigen or by incubating with a mitogen capable of inducing an immune response by the T-lymphocyte cells, following which said cells have been pressure treated by means of hydrostatic pressure in the order of 50.7 to 152 MPa (500 to 1500 atm) for sufficient time to cause augmented antigenicity of the T-lymphocyte cells without substantial loss of membrane proteins from the cells, wherein said specific activated cells have been cross-linked by means of a chemical cross-linking agent either before or after the pressure treatment.

31. A preparation for preventing or treating an autoimmune disease, comprising T-lymphocyte cells which have developed specificity for the autoimmune disease specific antigen and which cells have been activated either by incubating in the presence of the autoimmune disease specific antigen or by incubating with a mitogen capable of inducing an immune response by the T-lymphocyte cells, following which said cells have been pressure treated by means of hydrostatic pressure in the order of 50.7 to 152 MPa (500 to 1500 atm) for sufficient time to cause augmented antigenicity of the T-lymphocyte cells without substantial loss of membrane proteins from the cells, wherein said specific activated cells have had their cytoskeleton disrupted by means of a cytoskeletal disrupting agent either before or after the pressure treatment.

32. A preparation for preventing or treating an autoimmune disease, comprising T-lymphocyte cells which have developed specificity for the autoimmune disease specific antigen and which cells have been activated either by incubating in the presence of the autoimmune disease specific antigen or by incubating with a mitogen capable of inducing an immune response by the T-lymphocyte cells, following which said cells have been cross-linked by means of a chemical cross-linking agent.

33. A preparation for preventing or treating an autoimmune disease, comprising T-lymphocyte cells which have developed specificity for the autoimmune disease specific antigen and which cells have been activated either by incubating in the presence of the autoimmune disease specific antigen or by incubating with a mitogen capable of inducing an immune response by the T-lymphocyte cells, following which said cells have had their cytoskeletons disrupted by means of a cytoskeletal disrupting agent.

**Patentansprüche**

1. Druckbehandelte, aktivierte T-Lymphozytenzelle mit der Eignung zur Verhinderung oder Behandlung einer speziellen Autoimmunerkrankung, die mittels eines hydrostatischen Drucks in der Größenordnung von 50,7 - 152 MPa (500 bis 1500 atm) eine ausreichende Zeit lang druckbehandelt wurde, um eine vermehrte Antigenizität der T-Lymphozytenzellen ohne nennenswerten Verlust an Membranproteinen von den Zellen herbeizuführen, und die eine zusätzliche Behandlung mit einem chemischen Vernetzungsmittel erfahren hat.

2. Druckbehandelte, aktivierte T-Lymphozytenzelle nach Anspruch 1, dadurch gekennzeichnet, daß es sich bei der speziellen Autoimmunerkrankung um multiple Sklerose, Thyroiditis, Diabetes Typ I, ankylosierende Spondylitis, rheumatische Arthritis oder Myasthenia gravis handelt.

3. Druckbehandelte, aktivierte T-Lymphozytenzelle nach Anspruch 1, die nach der Druckbehandlung mit dem chemischen Vernetzungsmittel behandelt wurde.

4. Druckbehandelte, aktivierte T-Lymphozytenzelle nach Anspruch 1, die vor der Druckbehandlung mit dem chemischen Vernetzungsmittel behandelt wurde.

5. Druckbehandelte, aktivierte T-Lymphozytenzelle mit der Eignung zur Verhinderung oder Behandlung einer speziellen Autoimmunerkrankung, die mittels eines hydrostatischen Drucks in der Größenordnung von 50,7 - 152 MPa (500 bis 1500 atm) eine ausreichende Zeit lang druckbehandelt wurde, um eine vermehrte Antigenizität der T-Lymphozytenzellen ohne nennenswerten Verlust an Membranproteinen

EP 0 261 648 B1

von den Zellen herbeizuführen, und die einer zusätzlichen Behandlung mit einem Mittel zum Aufbrechen des Zellskeletts unterworfen wurde.

6. Druckbehandelte, aktivierte T-Lymphozytenzelle nach Anspruch 5, die nach der Druckbehandlung einer Behandlung mit dem Mittel zum Aufbrechen des Zellskeletts unterworfen wurde.

7. Druckbehandelte, aktivierte T-Lymphozytenzelle nach Anspruch 5, die vor der Druckbehandlung mit dem Mittel zum Aufbrechen des Zellskeletts behandelt wurde.

8. Mit einem chemischen Vernetzungsmittel behandelte, aktivierte T-Lymphozytenzelle mit der Eignung zur Verhinderung oder Behandlung einer speziellen Autoimmunerkrankung.

9. Mit einem chemischen Vernetzungsmittel behandelte, aktivierte T-Lymphozytenzelle nach Anspruch 8, dadurch gekennzeichnet, daß es sich bei der speziellen Autoimmunerkrankung um multiple Sklerose, Thyroiditis, Diabetes Typ I, ankylosierende Spondylitis, rheumatische Arthritis oder Myasthenia gravis handelt.

10. Mit einem Mittel zum Aufbrechen behandelte, aktivierte T-Lymphozytenzelle mit der Eignung zur Verhinderung oder Behandlung einer speziellen Autoimmunerkrankung.

11. Mit einem Mittel zum Aufbrechen behandelte, aktivierte T-Lymphozytenzelle nach Anspruch 10, dadurch gekennzeichnet, daß es sich bei der speziellen Autoimmunerkrankung um multiple Sklerose, Thyroiditis, Diabetes Typ I, ankylosierende Spondylitis, rheumatische Arthritis oder Myasthenia gravis handelt.

12. Mit einem Mittel zum Aufbrechen behandelte aktivierte T-Lymphozytenzelle nach Anspruch 10, die eine zusätzliche Behandlung mit einem chemischen Vernetzungsmittel erfahren hat.

13. Verfahren zur Herstellung druckbehandelter, aktivierter T-Lymphozyten mit der Eignung zur Verhinderung oder Behandlung einer speziellen Autoimmunerkrankung nach Anspruch 1, durch:
a) Suspendieren für die Autoimmunerkrankung spezifischer, aktivierter T-Lymphozytenzellen in einem Puffer;
b) Einwirkenlassen eines chemischen Vernetzungsmittels auf die suspendierten Zellen;
c) ausreichend langes Einwirkenlassen eines geeigneten hydrostatischen Drucks in der Größenordnung von 50,7 bis 152 MPa (500 bis 1500 atm) auf die behandelten Zellen zur Herbeiführung einer vermehrten Antigenizität der T-Lymphozytenzellen ohne merklichen Verlust an Membranproteinen von den Zellen und
d) Entspannen des auf die suspendierten Zellen einwirkenden Drucks mit geeigneter Geschwindigkeit zur Gewinnung druckbehandelter, aktivierter T-Lymphozytenzellen ohne merklichen Verlust an Membranproteinen von den Zellen.

14. Verfahren nach Anspruch 13, dadurch gekennzeichnet, daß Zellen zusätzlich mit einem Mittel zum Aufbrechen behandelt werden.

15. Verfahren zur Herstellung druckbehandelter, aktivierter T-Lymphozyten mit der Eignung zur Verhinderung oder Behandlung einer speziellen Autoimmunerkrankung nach Anspruch 1, durch:
a) Suspendieren für die Autoimmunerkrankung spezifischer, aktivierter T-Lymphozytenzellen in einem Puffer;
b) ausreichend langes Einwirkenlassen eines geeigneten hydrostatischen Drucks in der Größenordnung von 50,7 bis 152 MPa (500 bis 1500 atm) auf die suspendierten Zellen zur Herbeiführung einer vermehrten Antigenizität der T-Lymphozytenzellen ohne merklichen Verlust an Membranproteinen von den Zellen,
c) Entspannen des auf die suspendierten Zellen einwirkenden Drucks mit geeigneter Geschwindigkeit zur Gewinnung druckbehandelter, aktivierter T-Lymphozytenzellen ohne merklichen Verlust an Membranproteinen von den Zellen und
d) Einwirkenlassen eines chemischen Vernetzungsmittels auf die druckbehandelten Zellen.

16. Verfahren nach Anspruch 15, dadurch gekennzeichnet, daß die Zellen eine zusätzliche Behandlung mit

24

einem Mittel zum Aufbrechen erfahren.

17. Verfahren zur Herstellung druckbehandelter, aktivierter T-Lymphozyten mit der Eignung zur Verhinderung oder Behandlung einer speziellen Autoimmunerkrankung nach Anspruch 5, durch:

a) Suspendieren für die Autoimmunerkrankung spezifischer, aktivierter T-Lymphozytenzellen in einem Puffer;
b) Einwirkenlassen eines Mittels zum Aufbrechen des Zellskeletts auf die suspendierten Zellen;
c) ausreichend langes Einwirkenlassen eines geeigneten hydrostatischen Drucks in der Größenordnung von 50,7 bis 152 MPa (500 bis 1500 atm) auf die behandelten Zellen zur Herbeiführung einer vermehrten Antigenizität der T-Lymphozytenzellen ohne merklichen Verlust an Membranproteinen von den Zellen und
d) Entspannen des auf die suspendierten Zellen einwirkenden Drucks mit geeigneter Geschwindigkeit zur Gewinnung druckbehandelter, aktivierter T-Lymphozytenzellen ohne merklichen Verlust an Membranproteinen von den Zellen.

18. Verfahren zur Herstellung druckbehandelter, aktivierter T-Lymphozyten mit der Eignung zur Verhinderung oder Behandlung einer speziellen Autoimmunerkrankung nach Anspruch 5, durch:

a) Suspendieren für die Autoimmunerkrankung spezifischer, aktivierter T-Lymphozytenzellen in einem Puffer;
b) ausreichend langes Einwirkenlassen eines geeigneten hydrostatischen Drucks in der Größenordnung von 50,7 bis 152 MPa (500 bis 1500 atm) auf die suspendierten Zellen zur Herbeiführung einer vermehrten Antigenizität der T-Lymphozytenzellen ohne merklichen Verlust an Membranproteinen von den Zellen,
c) Entspannen des auf die suspendierten Zellen einwirkenden Drucks mit geeigneter Geschwindigkeit zur Gewinnung druckbehandelter, aktivierter T-Lymphozytenzellen ohne merklichen Verlust an Membranproteinen von den Zellen und
d) Einwirkenlassen eines Mittels zum Aufbrechen des Zellskeletts auf die druckbehandelten Zellen.

19. Verfahren zur Herstellung von mit einem chemischen Vernetzungsmittel behandelten, aktivierten T-Lymphozyten mit der Eignung zur Verhinderung oder Behandlung einer speziellen Autoimmunerkrankung durch:

a) Suspendieren für die Autoimmunerkrankung spezifischer, aktivierter T-Lymphozytenzellen in einem Puffer und
b) Behandeln der suspendierten Zellen mit einem chemischen Vernetzungsmittel.

20. Verfahren zur Herstellung von mit einem Mittel zum Aufbrechen behandelten, aktivierten T-Lymphozytenzellen mit der Eignung zur Verhinderung oder Behandlung einer speziellen Autoimmunerkrankung durch:

a) Suspendieren für die Autoimmunerkrankung spezifischer, aktivierter T-Lymphozytenzellen in einem Puffer und
b) Behandeln der suspendierten Zellen mit einem Mittel zum Aufbrechen zur Herstellung aktivierter T-Lymphozytenzellen mit aufgespaltenen Zellskeletten.

21. Verfahren nach Anspruch 20, dadurch gekennzeichnet, daß im Anschluß an Stufe b) die aktivierten T-Lymphozytenzellen mit aufgespaltenen Zellskeletten mit einem chemischen Vernetzungsmittel behandelt werden.

22. Mittel zur Verhinderung oder Behandlung einer speziellen Autoimmunerkrankung, umfassend druckbehandelte, mit einem Vernetzungsmittel behandelte, aktivierte T-Lymphozytenzellen nach Anspruch 1 und einen pharmazeutisch akzeptablen Träger.

23. Mittel zur Verhinderung oder Behandlung einer speziellen Autoimmunerkrankung, umfassend mit einem chemischen Vernetzungsmittel behandelte, aktivierte T-Lymphozytenzellen nach Anspruch 8 und einen pharmazeutisch akzeptablen Träger.

24. Mittel zur Verhinderung oder Behandlung einer speziellen Autoimmunerkrankung, umfassend mit einem Mittel zum Aufbrechen behandelte, aktivierte T-Lymphozytenzellenmembranfragmente nach Anspruch 10 und einen pharmazeutisch akzeptablen Träger.

**25.** Verfahren zur Gewinnung gereinigter Membranproteine aus aktivierten T-Lymphozytenzellen mit der Eignung zur Verhinderung oder Behandlung einer speziellen Autoimmunerkrankung, durch:

a) Suspendieren für die Autoimmunerkrankung spezifischer, aktivierter T-Lymphozytenzellen in einem Puffer;

b) Einwirkenlassen eines chemischen Vernetzungsmittels auf die suspendierten Zellen;

c) ausreichend langes Einwirkenlassen eines geeigneten hydrostatischen Drucks in der Größenordnung von 50,7 bis 152 MPa (500 bis 1500 atm) auf die behandelten Zellen zur Herbeiführung einer vermehrten Antigenizität der T-Lymphozytenzellen ohne merklichen Verlust an Membranproteinen von den Zellen;

d) Entspannen des auf die suspendierten Zellen einwirkenden Drucks mit bestimmter Geschwindigkeit;

e) Zentrifugieren der entspannten Suspension zur Entfernung von Zellfragmenten und zur Herstellung eines Überstands mit den Membranproteinen und

f) Behandeln des Überstands mittels einer Ultrazentrifuge zur Gewinnung der gereinigten Membranproteine.

**26.** Verfahren zur Gewinnung gereinigter Membranproteine aus aktivierten T-Lymphozytenzellen mit der Eignung zur Verhinderung oder Behandlung einer speziellen Autoimmunerkrankung, durch:

a) Suspendieren für die Autoimmunerkrankung spezifischer, aktivierter T-Lymphozytenzellen in einem Puffer;

b) ausreichend langes Einwirkenlassen eines geeigneten hydrostatischen Drucks in der Größenordnung von 50,7 bis 152 MPa (500 bis 1500 atm) auf die suspendierten Zellen zur Herbeiführung einer vermehrten Antigenizität der T-Lymphozytenzellen ohne merklichen Verlust an Membranproteinen von den Zellen;

c) Entspannen des auf die suspendierten Zellen einwirkenden Drucks mit bestimmter Geschwindigkeit;

d) Einwirkenlassen eines chemischen Vernetzungsmittels auf die druckbehandelten Zellen;

e) Zentrifugieren der entspannten Suspension zur Entfernung von Zellfragmenten und zur Herstellung eines Überstands mit den Membranproteinen und

f) Behandeln des Überstands mittels einer Ultrazentrifuge zur Gewinnung der gereinigten Membranproteine.

**27.** Verfahren zur Gewinnung gereinigter Membranproteine aus aktivierten T-Lymphozytenzellen mit der Eignung zur Verhinderung oder Behandlung einer speziellen Autoimmunerkrankung, durch:

a) Suspendieren für die Autoimmunerkrankung spezifischer, aktivierter T-Lymphozytenzellen in einem Puffer;

b) Einwirkenlassen eines Mittels zum Aufbrechen des Zellskeletts auf die suspendierten Zellen;

c) ausreichend langes Einwirkenlassen eines geeigneten hydrostatischen Drucks in der Größenordnung von 50,7 bis 152 MPa (500 bis 1500 atm) auf die behandelten Zellen zur Herbeiführung einer vermehrten Antigenizität der T-Lymphozytenzellen ohne merklichen Verlust an Membranproteinen von den Zellen;

d) Entspannen des auf die suspendierten Zellen einwirkenden Drucks mit bestimmter Geschwindigkeit;

e) Zentrifugieren der entspannten Suspension zur Entfernung von Zellfragmenten und zur Herstellung eines Überstands mit den Membranproteinen und

f) Behandeln des Überstands mittels einer Ultrazentrifuge zur Gewinnung der gereinigten Membranproteine.

**28.** Verfahren zur Gewinnung gereinigter Membranproteine aus aktivierten T-Lymphozytenzellen mit der Eignung zur Verhinderung oder Behandlung einer speziellen Autoimmunerkrankung, durch:

a) Suspendieren für die Autoimmunerkrankung spezifischer, aktivierter T-Lymphozytenzellen in einem Puffer;

b) ausreichend langes Einwirkenlassen eines geeigneten hydrostatischen Drucks in der Größenordnung von 50,7 bis 152 MPa (500 bis 1500 atm) auf die suspendierten Zellen zur Herbeiführung einer vermehrten Antigenizität der T-Lymphozytenzellen ohne merklichen Verlust an Membranproteinen von den Zellen;

c) Entspannen des auf die suspendierten Zellen einwirkenden Drucks mit bestimmter Geschwindigkeit;

d) Einwirkenlassen eines Mittels zum Aufbrechen des Zellskeletts auf die druckbehandelten Zellen;

e) Zentrifugieren der entspannten Suspension zur Entfernung von Zellfragmenten und zur Herstellung eines Überstands mit den Membranproteinen und

f) Behandeln des Überstands mittels einer Ultrazentrifuge zur Gewinnung der gereinigten Membranproteine.

29. Verfahren zur Gewinnung gereinigter Membranproteine aus aktivierten T-Lymphozytenzellen mit der Eignung zur Verhinderung oder Behandlung einer speziellen Autoimmunerkrankung, durch:

a) Suspendieren für die Autoimmunerkrankung spezifischer, aktivierter T-Lymphozytenzellen in einem Puffer;

b) Behandeln des Überstands mittels einer Ultrazentrifuge zur Gewinnung der gereinigten Membranproteine;

c) ausreichend langes Einwirkenlassen eines geeigneten hydrostatischen Drucks in der Größenordnung von 50,7 bis 152 MPa (500 bis 1500 atm) auf die suspendierten Zellen zur Herbeiführung einer vermehrten Antigenizität der T-Lymphozytenzellen ohne merklichen Verlust an Membranproteinen aus den Zellen;

d) Entspannen des auf die suspendierten Zellen einwirkenden Drucks mit bestimmter Geschwindigkeit;

e) Zentrifugieren der entspannten Suspension zur Entfernung von Zellfragmenten und zur Herstellung eines Überstands mit den Membranproteinen und

f) Einwirkenlassen eines chemischen Vernetzungsmittels auf die "abgestoßenen" Membranproteine.

30. Präparat zur Verhinderung oder Behandlung einer Autoimmunerkrankung, umfassend T-Lymphozytenzellen mit entwickelter Spezifität für das für die Autoimmunerkrankung spezifische Antigen, die entweder durch Inkubieren in Gegenwart des für die Autoimmunerkrankung spezifischen Antigens oder durch Inkubieren mit einem Mitogen mit der Fähigkeit zur Induktion einer Immunantwort durch die T-Lymphozytenzellen aktiviert und dann eine ausreichende Zeit lang mittels eines hydrostatischen Drucks in der Größenordnung von 50,7 bis 152 MPa (50 bis 1500 atm) druckbehandelt wurden, um eine vermehrte Antigenizität der T-Lymphozytenzellen ohne merklichen Verlust an Membranproteinen aus den Zellen herbeizuführen, wobei die spezifischen aktivierten Zellen mit Hilfe eines chemischen Vernetzungsmittels entweder vor oder nach der Druckbehandlung vernetzt wurden.

31. Präparat zur Verhinderung oder Behandlung einer Autoimmunerkrankung, umfassend T-Lymphozytenzellen mit entwickelter Spezifität für das für die Autoimmunerkrankung spezifische Antigen, die entweder durch Inkubieren in Gegenwart des für die Autoimmunerkrankung spezifischen Antigens oder durch Inkubieren mit einem Mitogen mit der Fähigkeit zur Induktion einer Immunantwort durch die T-Lymphozytenzellen aktiviert und dann eine ausreichende Zeit lang mittels eines hydrostatischen Drucks in der Größenordnung von 50,7 bis 152 MPa (50 bis 1500 atm) druckbehandelt wurden, um eine vermehrte Antigenizität der T-Lymphozytenzellen ohne merklichen Verlust an Membranproteinen aus den Zellen herbeizuführen, wobei das Zellskelett der spezifischen aktivierten Zellen mit Hilfe eines Mittels zum Aufbrechen des Zellskeletts-entweder vor oder nach der Druckbehandlung aufgebrochen wurde.

32. Präparat zur Verhinderung oder Behandlung einer Autoimmunerkrankung, umfassend T-Lymphozytenzellen mit entwickelter Spezifität für das für die Autoimmunerkrankung spezifische Antigen, die entweder durch Inkubieren in Gegenwart des für die Autoimmunerkrankung spezifischen Antigens oder durch Inkubieren mit einem Mitogen mit der Fähigkeit zur Induktion einer Immunantwort durch die T-Lymphozytenzellen aktiviert und dann mit Hilfe eines chemischen Vernetzungsmittels vernetzt wurden.

33. Präparat zur Verhinderung oder Behandlung einer Autoimmunerkrankung, umfassend T-Lymphozytenzellen mit entwickelter Spezifität für das für die Autoimmunerkrankung spezifische Antigen, die entweder durch Inkubieren in Gegenwart des für die Autoimmunerkrankung spezifischen Antigens oder durch Inkubieren mit einem Mitogen mit der Fähigkeit zur Induktion einer Immunantwort durch die T-Lymphozytenzellen aktiviert und deren Zellskelette danach mit Hilfe eines Mittels zum Aufbrechen des Zellskeletts aufgebrochen wurden.

**Revendications**

**EP 0 261 648 B1**

1. Cellule de lymphocyte T activée, traitée sous pression, utile pour prévenir ou traiter une maladie autoimmune spécifique, ayant été traitée sous pression au moyen d'une pression hydrostatique de l'ordre de 50,7 à 152 MPa (500 à 1.500 atm) pendant un laps de temps suffisant pour provoquer une augmentation d'antigénicité des cellules de lymphocytes T sans perte importante des protéines des membranes de ces cellules, et qui a été traitée en outre avec un agent chimique de réticulation.

2. Cellule de lymphocyte T activée, traitée sous pression, selon la revendication 1, caractérisée en ce que l'affection spécifique autoimmune est la sclérose multiple, la thyroïdite, le diabète type I, la spondylite ankylosante, l'arthrite rhumatoïde, ou la myasthénia gravis.

3. Cellule de lymphocyte T activée, traitée sous pression, selon la revendication 1, qui a été traitée avec l'agent chimique de réticulation après avoir été traitée sous pression.

4. Cellule de lymphocyte T activée, traitée sous pression, selon la revendication 1, qui a été traitée avec l'agent chimique de réticulation avant d'être traitée sous pression.

5. Cellule de lymphocyte T activée, traitée sous pression, utile pour prévenir ou traiter une affection spécifique autoimmune, ayant été traitée sous pression au moyen d'une pression hydrostatique de l'ordre de 50,7 à 152 MPa (500 à 1.500 atm) pendant un laps de temps suffisant pour provoquer une augmentation d'antigénicité des cellules de lymphocytes T sans perte importante des protéines des membranes de ces cellules, et qui a été traitée en outre avec un agent qui provoque la rupture de son cytosquelette.

6. Cellule de lymphocyte T activée, traitée sous pression, selon la revendication 5, qui a été traitée avec l'agent de rupture après avoir été traitée sous pression.

7. Cellule de lymphocyte T activée, traitée sous pression, selon la revendication 5, qui a été traitée avec l'agent de rupture avant d'être traitée sous pression.

8. Cellule de lymphocyte T activée, traitée avec un agent chimique de réticulation, utile pour prévenir ou traiter une affection autoimmune spécifique.

9. Cellule de lymphocyte T activée, traitée avec un agent chimique de réticulation, selon la revendication 8, caractérisée en ce que l'affection autoimmune est la sclérose multiple, la thyroïdite, le diabète type I, la spondylite ankylosante, l'arthrite rhumatoïde, ou la myasthenia gravis.

10. Cellule de lymphocyte T activée, traitée avec un agent de rupture, utile pour prévenir ou traiter une affection autoimmune spécifique.

11. Cellule de lymphocyte T activée, traitée avec un agent de rupture, selon la revendication 10, caractérisée en ce que l'affection autoimmune spécifique est la sclérose multiple, la thyroïdite, le diabète type I, la spondylite ankylosante, l'arthrite rhumatoïde, ou la myasthenia gravis.

12. Cellule de lymphocyte T activée, traitée avec un agent de rupture, selon la revendication 10, qui a été traitée en outre avec un agent chimique de réticulation.

13. Procédé pour produire des lymphocytes T activés, traités sous pression, utiles pour prévenir ou traiter une affection autoimmune spécifique, selon la revendication 1, caractérisé en ce qu'il comporte les phases opératoires suivantes :
    a) on met en suspension des cellules de lymphocytes T activées, spécifiques de l'affection autoimmune, dans une solution tampon ;
    b) on soumet les cellules en suspension à un agent chimique de réticulation ;
    c) on soumet les cellules traitées à une pression hydrostatique appropriée de l'ordre de 50,7 à 152 MPa (500 à 1.500atm) pendant un laps de temps suffisant pour provoquer une augmentation de l'antigénicité des cellules de lymphocytes T sans perte importante des protéines des membranes de ces cellules ;
    d) et on fait chuter la pression appliquée aux cellules en suspension, en opérant à un taux de réduction approprié pour produire des cellules de lymphocytes T activées, traitées sous pression,

28

EP 0 261 648 B1

sans perte importante des protéines des membranes de ces cellules.

14. Procédé selon la revendication 13, caractérisé en ce qu'on traite en outre les cellules avec un agent de rupture.

15. Procédé pour produire des lymphocytes T activées, traités sous pression, utiles pour prévenir ou traiter une affection autoimmune spécifique, selon la revendication 1, caractérisé en ce qu'il comporte les phases opératoires suivantes :
   a) on met en suspension des cellules de lymphocytes T activées, spécifiques de l'affection autoimmune, dans une solution tampon ;
   b) on soumet ces cellules en suspension à une pression hydrostatique appropriée de l'ordre de 50,7 à 152 MPa (500 à 1.500 atm) pendant un laps de temps suffisant pour provoquer une augmentation de l'antigénicité des cellules de lymphocytes T sans perte importante des protéines des membranes des cellules ;
   c) on fait chuter la pression appliquée aux cellules en suspension, en opérant à un taux de réduction approprié pour produire des cellules de lymphocytes T activées, traitées sous pression, sans perte importante des protéines des membranes de ces cellules ;
   d) et on soumet ces cellules traitées sous pression à un agent chimique de réticulation.

16. Procédé selon la revendication 15, caractérisé en ce qu'on traite en outre les cellules avec un agent de rupture.

17. Procédé pour produire des lymphocytes T activés, utiles pour prévenir ou traiter une affection autoimmune spécifique, selon la revendication 5, caractérisé en ce qu'il comporte les phases opératoires suivantes :
   a) on met en suspension des cellules de lymphocytes T activés, spécifiques de l'affection autoimmune, dans une solution tampon ;
   b) on soumet ces cellules en suspension à un agent de rupture du cytosquelette ;
   c) on soumet les cellules ainsi traitées à une pression hydrostatique appropriée de l'ordre de 50,7 à 152 MPa (500 à 1.500 atm) pendant un laps de temps suffisant pour provoquer une augmentation de l'antigénicité des cellules de lymphocytes T sans perte importante des protéines des membranes des cellules ;
   d) on fait chuter la pression appliquée aux cellules en suspension, en opérant à un taux de réduction approprié pour produire des cellules de lymphocytes T activées, traitées sous pression, sans perte importante des protéines des membranes des cellules.

18. Procédé pour produire des lymphocytes T activés, traités sous pression, utiles pour prévenir ou traiter une affection autoimmune spécifique, selon la revendication 5, caractérisé en ce qu'il comporte les phases opératoires suivantes :
   a) on met en suspension des cellules de lymphocytes T activés, spécifiques de l'affection autoimmune, dans une solution tampon ;
   b) on soumet ces cellules en suspension à une pression hydrostatique appropriée de l'ordre de 50,7 à 152 MPa (500 à 1.500 atm) pendant un laps de temps suffisant pour provoquer une augmentation de l'antigénicité des cellules de lymphocytes T sans perte importante des protéines des membranes des cellules ;
   c) on fait chuter la pression appliquée sur les cellules en opérant à un taux de réduction approprié pour produire des cellules de lymphocytes T activées, traitées sous pression, sans perte importante des protéines des membranes de ces cellules ;
   d) et on soumet ces cellules traitées sous pression à un agent de rupture de leur cytosquelette.

19. Procédé pour produire des cellules de lymphocytes T activées, traitées avec un agent chimique de réticulation, utiles pour prévenir ou traiter une affection autoimmune spécifique, caractérisé en ce qu'il comporte les phases opératoires suivantes :
   a) on met en suspension des cellules de lymphocytes T activées, spécifiques de l'affection autoimmune, dans une solution tampon ;
   b) et on traite ces cellules en suspension avec un agent chimique de réticulation.

20. Procédé pour produire des cellules de lymphocytes T activées, traitées avec un agent de rupture, utiles

29

pour prévenir ou traiter une affection autoimmune spécifique, caractérisé en ce qu'il comporte les phases opératoires suivantes :

a) on met en suspension des cellules de lymphocytes T activées, spécifiques de l'affection autoimmune, dans une solution tampon ;

b) et on traite les cellules en suspension avec un agent de rupture pour produire des cellules de lymphocytes T activées dont le cytosquelette est dissocié.

21. Procédé selon la revendication 20, caractérisé en ce que, après la phase b), on traite les cellules de lymphocytes T activées dont le squelette est dissocié, par un agent chimique de réticulation.

22. Composition pour prévenir ou traiter une affection autoimmune spécifique qui comporte des cellules de lymphocytes T activées, traitées sous pression, et traitées avec un agent de réticulation, conformes à la revendication 1, et un support acceptable du point de vue pharmaceutique.

23. Composition pour prévenir ou traiter une affection autoimmune spécifique qui comporte des cellules de lymphocytes T activées, traitées avec un agent chimique de réticulation, conformes a la revendication 8, et un support acceptable du point de vue pharmaceutique.

24. Composition pour prévenir ou traiter une affection autoimmune spécifique qui comporte des fragments de membranes de cellules de lymphocytes T activées, traitées avec un agent de rupture, conformes à la revendication 10, et un support acceptable du point de vue pharmaceutique.

25. Procédé pour récupérer des protéines purifiées provenant des membranes de cellules de lymphocytes T activées, utiles pour prévenir ou traiter une affection autoimmune spécifique, caractérisé en ce qu'il comporte les phases opératoires suivantes :

a) on met en suspension des cellules de lymphocytes T activées, spécifiques de l'affection autoimmune, dans une solution tampon ;

b) on soumet ces cellules en suspension à un agent chimique de réticulation ;

c) on soumet les cellules ainsi traitées à une pression hydrostatique appropriée , de l'ordre de 50,7 à 152 MPa (500 à 1.500 atm), pendant un laps de temps suffisant pour provoquer une augmentation de l'antigénicité des cellules de lymphocytes T, sans perte importante des protéines des membranes de ces cellules ;

d) on fait chuter la pression appliquée sur les cellules en suspension, en opérant à un taux de réduction approprié ;

e) on traite par centrifugation la suspension ramenée à la pression ambiante pour éliminer les fragments des cellules et obtenir un produit qui surnage et qui contient les protéines des membranes ;

f) on traite par ultracentrifugation ce produit surnageant pour obtenir les protéines purifiées des membranes.

26. Procédé pour obtenir des protéines purifiées provenant des membranes de cellules de lymphocytes T activées utiles pour prévenir ou traiter une affection autoimmune spécifique, caractérisé en ce qu'il comporte les phases opératoires suivantes :

a) on met en suspension des cellules de lymphocytes T activées, spécifiques de l'affection autoimmune, dans une solution tampon ;

b) on soumet ces cellules en suspension à une pression hydrostatique appropriée, de l'ordre de 50,7 à 152 MPa (500 à 1.500 atm), pendant un laps de temps suffisant pour provoquer une augmentation de l'antigénicité des cellules de lymphocytes T, sans perte importante des protéines des membranes de ces cellules ;

c) on fait chuter la pression appliquée à ces cellules en suspension, en opérant à un taux de réduction approprié ;

d) on soumet les cellules ainsi traitées sous pression à un agent chimique de réticulation ;

e) on traite par centrifugation la suspension ramenée à la pression ambiante pour éliminer les fragments des cellules et obtenir un produit qui surnage et qui contient les protéines des membranes ;

f) et on traite par ultracentrifugation ce produit surnageant pour obtenir les protéines purifiées des membranes.

**27.** Procédé pour obtenir des protéines purifiées provenant des membranes de cellules de lymphocytes T activées utiles pour prévenir ou traiter une affection autoimmune spécifique, caractérisé en ce qu'il comporte les phases opératoires suivantes :

a) on met en suspension des cellules de lymphocytes T activées spécifiques de l'affection autoimmune, dans une solution tampon ;

b) on soumet les cellules en suspension à un agent de rupture de leur cytosquelette ;

c) on soumet les cellules ainsi traitées à une pression hydrostatique appropriée, de l'ordre de 50,7 à 152 MPa (500 à 1.500 atm), pendant un laps de temps suffisant pour provoquer une augmentation de l'antigénicité des cellules de lymphocytes T sans perte importante des protéines des membranes des cellules ;

d) on fait chuter la pression appliquée aux cellules en suspension, en opérant à un taux de réduction approprié ;

e) on traite par centrifugation la suspension ramenée à la pression ambiante pour éliminer les fragments des cellules et obtenir un produit qui surnage et qui contient les protéines des membranes ;

f) on traite par ultracentrifugation ce produit qui a surnagé pour recueillir les protéines purifiées des membranes.

**28.** Procédé pour obtenir des protéines purifiées provenant des membranes de cellules de lymphocytes T activées, utiles pour prévenir ou traiter une affection autoimmune spécifique, caractérisé en ce qu'il comporte les phases opératoires suivantes :

a) on met en suspension des cellules de lymphocytes T activées, spécifiques de l'affection autoimmune, dans une solution tampon ;

b) on soumet ces cellules en suspension à une pression hydrostatique appropriée, de l'ordre de 50,7 à 152 MPa (500 à 1.500 atm), pendant un laps de temps suffisant pour provoquer une augmentation de l'antigénicité des cellules de lymphocytes T sans perte importante des protéines des membranes de ces cellules ;

c) on fait chuter la pression appliquée à ces cellules en suspension, en opérant à un taux de réduction approprié ;

d) on soumet les cellules ainsi traitées sous pression à un agent de rupture de leur cytosquelette ;

e) on traite par centrifugation la suspension ramenée à la pression ambiante pour éliminer les fragments des cellules et obtenir un produit qui surnage et qui contient les protéines des membranes ;

f) et on traite par ultracentrifugation ce produit qui a surnagé pour obtenir les protéines purifiées des membranes.

**29.** Procédé pour obtenir des protéines purifiées provenant des membranes de cellules de lymphocytes T activées, utiles pour prévenir ou traiter une affection autoimmune spécifique, caractérisé en ce qu'il comporte les phases opératoires suivantes :

a) on met en suspension des cellules de lymphocytes T activées, spécifiques de l'affection autoimmune, dans une solution tampon ;

b) on traite par ultracentrifugation ce produit qui a surnage pour recueillir les protéines purifies des membranes ;

c) on soumet ces cellules en suspension à une pression hydrostatique appropriée, de l'ordre de 50,7 à 152 MPa (500 à 1.500 atm), pendant un laps de temps suffisant pour augmenter l'antigénicité des cellules de lymphocytes T, sans perte importante des protéines des membranes des cellules ;

d) on fait chuter la pression appliquée aux cellules en suspension, en opérant à un taux de réduction approprié ;

e) on traite par centrifugation la suspension ramenée à la pression ambiante pour éliminer les fragments des cellules et obtenir un produit qui surnage et qui contient les protéines des membranes ;

f) et on soumet les protéines ainsi séparées des membranes à un agent chimique de réticulation.

**30.** Préparation pour prévenir ou traiter une affection autoimmune spécifique, comportant des cellules de lymphocytes T qui ont acquis une spécificité pour l'antigène spécifique de l'affection autoimmune, ces cellules ayant été activées soit par incubation en présence de l'antigène spécifique de l'affection autoimmune, soit par incubation avec un mitogène capable d'induire une réponse d'immunité de la part des cellules de lymphocytes T, après quoi les cellules en question ont été traitées sous pression en leur appliquant une pression hydrostatique de l'ordre de 50,7 à 152 MPa (500 à 1.500 atm) pendant un laps de temps suffisant pour provoquer une augmentation de l'antigénicité des cellules de lymphocytes T sans perte importante des protéines des membranes des cellules, caractérisée en ce que les cellules activées spécifiques en question ont subi un traitement de réticulation avec un agent chimique de réticulation, soit avant soit après leur traitement sous pression.

31. Préparation pour prévenir ou traiter une affection autoimmune, comportant des cellules de lymphocytes T qui ont acquis une spécificité pour l'antigène spécifique de l'affection autoimmune, ces cellules ayant été activées, soit par incubation en présence de l'antigène spécifique de l'affection autoimmune, soit par incubation avec un mitogène capable d'induire une réponse d'immunité de la part de ces cellules de lymphocytes T, après quoi les cellules en question ont été traitées sous pression en leur appliquant une pression hydrostatique de l'ordre de 50,7 à 152 MPa (500 à 1.500 atm), pendant un laps de temps suffisant pour provoquer une augmentation de l'antigénicité des cellules de lymphocytes T, sans perte importante des protéines des membranes des cellules, caractérisée en ce que les cellules spécifiques activées en question ont eu leur cytosquelette brisé par un agent de rupture du cytosquelette, soit avant soit après leur traitement sous pression.

32. Préparation pour prévenir ou traiter une affection autoimmune, comportant des cellules de lymphocytes T qui ont acquis une spécificité pour l'antigène spécifique de l'affection autoimmune, ces cellules ayant été activées, soit par incubation en présence de l'antigène spécifique de l'affection autoimmune, soit par incubation avec un mitogène capable d'induire une réponse d'immunité de la part des cellules de lymphocytes T, après quoi ces cellules ont été réticulées avec un agent chimique de réticulation.

33. Préparation pour prévenir ou traiter une affection autoimmune, comportant des cellules de lymphocytes T qui ont acquis une spécificité pour l'antigène spécifique de l'affection autoimmune, ces cellules ayant été activées, soit par incubation en présence de l'antigène spécifique de l'affection autoimmune, soit par incubation avec un mitogène capable d'induire une réponse d'immunité de la part des cellules de lymphocytes T, après quoi les cellules en question ont eu leur cytosquelette brisé avec un agent de rupture du cytosquelette.

Figure 1

Figure 2

Figure 3

Figure 4

Figure 5